# EUROPEAN PATENT APPLICATION

(11) **EP 4 604 054 A1**
(43) Date of publication of application: **20.08.2025**
(21) Application number: 23893561.3
(22) Date of filing: 30.10.2023
(51) Int. Cl.: G06T 7/00, A61B 5/107

(54) **SPINE HEALTH RISK ASSESSMENT METHOD AND RELATED APPARATUS**

(30) Priority: 23.11.2022 CN 202211475517
(71) Applicant: Huawei Technologies Co., Ltd., Shenzhen, Guangdong 518129 (CN)
(72) Inventor: LU, Mu, Shenzhen, Guangdong 518129 (CN); SUN, Yu, Shenzhen, Guangdong 518129 (CN); MA, Chunhui, Shenzhen, Guangdong 518129 (CN); CHEN, Xiaohan, Shenzhen, Guangdong 518129 (CN); ZHAO, Jie, Shenzhen, Guangdong 518129 (CN)
(74) Representative: Gill Jennings & Every LLP
(86) International application number: PCT/CN2023/127913
(87) International publication number: WO 2024/109470

(57) **Abstract**

Embodiments of this application provide a spinal health risk assessment method. **In** this method, an electronic device may display an image captured by a camera, and then output guide information based on the image captured by the camera, where the guide information is used to prompt a user to adjust to a first state. After the image captured by the camera represents that the user is in the first state, the electronic device starts to shoot a photo or record a video, and determines and outputs an assessment result based on the photo or the video. **In** this way, the user can quickly and efficiently adjust a position and a posture, and a spinal health risk of the user is assessed by analyzing a shot photo or a recorded video. The method is simple and convenient, and a guiding process is complete, and the method can be used in a plurality of scenarios, so that operation difficulty of spinal health risk assessment is reduced, and convenience of spinal health risk assessment is improved.

## Description

This application claims priority to Chinese Patent Application No. 202211475517.7, filed with the China National Intellectual Property Administration on November 23, 2022, and entitled "SPINAL HEALTH RISK ASSESSMENT METHOD AND RELATED APPARATUS", which is incorporated herein by reference in its entirety.

### TECHNICAL FIELD

This application relates to the field of terminal technologies, and in particular, to a spinal health risk assessment method and a related apparatus.

### BACKGROUND

Scoliosis is one of common diseases of people and affects physical development and body shapes of people. In serious cases, cardiopulmonary functions and even spinal cord are affected, causing paralysis. The disease is insidious, has no obvious symptoms in the early stage, and is likely to be neglected, and therefore the optimal treatment time is missed. Therefore, it is of great significance to perform early screening of scoliosis.

In the conventional technology, severity of scoliosis is generally diagnosed by calculating a Cobb angle or an axial angle of trunk rotation (Angle of Trunk Rotation, ATR).

When the Cobb angle is used for diagnosis, upper and lower end vertebras are first determined. The upper and lower end vertebras are vertebral bodies with largest inclination to a concave side of scoliosis. A transverse line is drawn on an upper edge of the vertebral body of the upper end vertebra, and a transverse line is also drawn on a lower edge of the vertebral body of the lower end vertebra. Perpendicular lines are drawn for the two transverse lines respectively. An included angle between the three perpendicular lines is the Cobb angle. Although this method is more universal, the Cobb angle cannot be accurately calculated based on human body appearance data, and can be obtained only when additional invasive ray examination is performed to expose features of the entire spine. In routine screening, a forward flexion test is usually used to measure an angle of trunk rotation. A scoliosis measurement instrument may be used to measure back segments (thoracic, thoracolumbar, and lumbar segments) of a child and record a largest deflection angle that is found and a position. If the most serious asymmetry of the back exceeds 5°, scoliosis is highly suspected. The foregoing method has the following problems: Participation of a professional inspector is required, a measurement result may be affected by a subjective measurement method of a measurer and have an error, a magnitude of the error cannot be controlled, and an operation is relatively complex.

### SUMMARY

This application provides a spinal health risk assessment method and a related apparatus. A user may be guided to adjust a state, and after the user adjusts to a first state, a photo is shot or a video is recorded, to assess a spinal health risk of the user, thereby quickly and conveniently meeting an actual requirement of the user, and improving use experience of the user.

According to a first aspect, an embodiment of this application provides a spinal health risk assessment method. The method includes: An electronic device starts a camera, and captures an image by using the camera; the electronic device displays, on a first user interface, the image captured by the camera; the electronic device outputs guide information, where the guide information is used to prompt a user to adjust to a first state; after the image captured by the camera represents that the user is in the first state, the electronic device starts to shoot a photo or record a video; and the electronic device determines and outputs an assessment result based on the photo or the video.

According to the method provided in the first aspect, the guide information may be output to guide the user to adjust a state, to help the user quickly and efficiently adjust a position and a posture. After the user adjusts to the first state, an angle of trunk rotation of the user is determined by shooting a photo or recording a video, to assess a spinal health risk of the user. The method is simple and convenient, and a guiding process is complete, and the method can be used in a plurality of scenarios, so that operation difficulty of spinal health risk assessment is reduced, and convenience of spinal health risk assessment is improved. In this way, an actual requirement of the user can be quickly and conveniently met, thereby improving use experience of the user.

With reference to the first aspect, in some implementations, the guide information includes an interface element displayed by the electronic device on the first user interface, and/or a voice instruction output by the electronic device. Specifically, the interface element may be a text, a graph, a picture, and/or the like, so that the user can intuitively understand the guide information. Specifically, the guide information may display, in the interface element, "The current portrait deviates. Adjust the portrait position", "Face away from the camera", "Bend forward by 90 degrees", "Put both palms together", "Keep both legs apart at a distance the same as the shoulder width", and/or the like.

With reference to the first aspect, in some implementations, in a process in which the electronic device outputs the guide information, the electronic device displays a guide box on the first user interface, and the electronic device outputs first guide information, where the first guide information is used to prompt the user to adjust to the first position; and the electronic device outputs second guide information when the guide box displays a portrait captured by the camera, where the second guide information is used to prompt the user to adjust to the first posture. In this way, the user can adjust the state in time based on different guide information.

With reference to the first aspect, in some implementations, the electronic device adjusts a focal length of the camera based on a size of the portrait captured by the camera, and/or prompts the user to adjust a distance from the camera. The electronic device may zoom out the focal length when the portrait captured by the camera is excessively large, or zoom in the focal length when the portrait captured by the camera is excessively small. When the portrait captured by the camera is excessively large, the electronic device may prompt, in one or more forms, the user to stay away from the camera, and when the portrait captured by the camera is excessively small, prompt the user to approach the camera. The foregoing form may be a voice instruction and/or text information.

With reference to the first aspect, in some implementations, the electronic device may perform image analysis on the image captured by the camera, to determine whether the user is in the first state. Specifically, the electronic device may extract the portrait from the image captured by the camera, perform recognition by using a bone node algorithm, and analyze whether both legs of the user keep apart at a distance the same as a shoulder width, whether the body of the user is bent forward by 90 degrees, whether the back side of the user faces the camera, and whether both palms of the user are pressed together and whether tips of the hands center on both knees. In this way, whether the user is in the first state can be efficiently and conveniently determined.

With reference to the first aspect, in some implementations, after outputting the guide information, the electronic device may further determine a first distance and a second distance in the portrait captured by the camera, and the electronic device may determine a body forward flexion angle of the user based on a ratio of the first distance to the second distance, to determine whether the body forward flexion angle of the user is 90 degrees. The first distance and the second distance may be respectively a distance from a knee to an ankle, and a distance from a shoulder to a tip of a hand in both hands, or the first distance and the second distance may be respectively a distance from a hip to the ankle and a distance from the shoulder to the ankle. For example, when a front side of the user faces the camera, the electronic device may extract left and right shoulder nodes, left and right knee nodes, left and right hand tip nodes, and left and right ankle nodes based on the portrait captured by the camera. In this case, the first distance may be the distance from the knee to the ankle, and the second distance may be the distance from the shoulder to the tip of the hand in both hands. When the back side of the user faces the camera, the electronic device may extract left and right shoulder nodes, a hip node, left and right hand tip nodes, and left and right ankle nodes based on the portrait captured by the camera. In this case, the first distance may be the distance from the hip to the ankle, and the second distance may be the distance from the shoulder to the ankle.

With reference to the first aspect, in some implementations, the electronic device may determine, based on hand tip node positions and knee node positions in the portrait captured by the camera, whether both palms of the user are pressed together and whether hand tip positions center on both knees.

With reference to the first aspect, in some implementations, the electronic device may determine an angle of trunk rotation ATR of the user based on the photo or the video, further determine a spinal health status of the user, and then output the assessment result indicating the spinal health status of the user.

With reference to the first aspect, in some implementations, after the image captured by the camera represents that the user is in the first state, and before starting to shoot the photo, the electronic device outputs first prompt information, to prompt the user to keep the first state, so that a photo in which the user is in the first state exists in photos shot by the user.

With reference to the first aspect, in some implementations, after the image captured by the camera represents that the user is in the first state, and before starting to record a video, the electronic device outputs second prompt information, where the second prompt information is used to prompt the user to change from the first state to an upright state, or prompt the user to change from the upright state to the first state, so that an image in which the user is in the first state exists in the video recorded by the user.

With reference to the first aspect, in some implementations, before the electronic device starts to shoot the photo or record the video, the electronic device may receive a first operation, where the first operation is used to trigger the electronic device to shoot the photo or record the video. An implementation form of the first operation may be, for example, touching, by the user, a first control, or sending, by the user, a voice instruction for starting shooting or starting recording.

With reference to the first aspect, in some implementations, the electronic device may output the guide information when starting to display the first user interface. In another implementation, the electronic device may alternatively output the guide information after detecting that the portrait exists in the image captured by the camera.

With reference to the first aspect, in some implementations, the assessment result includes any one or more of the following: the ATR and a spinal health risk level, where a larger ATR indicates a higher corresponding spinal health risk level.

With reference to the first aspect, in some implementations, the electronic device may further output any one or more of the following: an exercise suggestion, a diet suggestion, a medical suggestion, and a living habit suggestion, and output a recommendation result of a sports game, and the like.

With reference to the first aspect, in some implementations, before the electronic device starts the camera and captures the image by using the camera, the electronic device may further display third prompt information, where the third prompt information is used to notify the user of related precautions, and the third prompt information may include a posture requirement for the user and a wearing requirement for the user.

According to a second aspect, an embodiment of this application provides an electronic device, including a memory and one or more processors, where the memory is coupled to the one or more processors, the memory is configured to store computer program code, the computer program code includes computer instructions, and the one or more processors invoke the computer instructions to enable the electronic device to perform:
starting a camera, and capturing an image by using the camera;
displaying, on a first user interface, the image captured by the camera;
outputting guide information, where the guide information is used to prompt a user to adjust to a first state;
after the image captured by the camera represents that the user is in the first state, starting to shoot a photo or record a video; and
determining and outputting an assessment result based on the photo or the video.

For a specific implementation of each component included in the electronic device in the second aspect, refer to the method described in the first aspect. Details are not described herein again.

According to a third aspect, an embodiment of this application provides an electronic device, including: a memory and one or more processors, where the memory is coupled to the one or more processors, the memory is configured to store computer program code, the computer program code includes computer instructions, and the one or more processors invoke the computer instructions to enable the electronic device to perform the method in any implementation performed by a first device side in the second aspect or the third aspect.

According to a fourth aspect, an embodiment of this application provides a computer-readable storage medium, including instructions. When the instructions are run on an electronic device, the electronic device is enabled to perform the method in the first aspect or any possible implementation of the first aspect.

According to a fifth aspect, an embodiment of this application provides a computer program product. When the computer program product is run on a computer, the computer is enabled to perform the method in the first aspect or any possible implementation of the first aspect.

According to the technical solution provided in this application, the electronic device may guide the user to adjust to the first state, and after determining, by using a preset strategy, that the user is in the first state, start to shoot a photo or record a video, calculate the angle of trunk rotation of the user from the shot photo or recorded video, and map the spinal health risk level based on the angle of trunk rotation, to output the corresponding spinal health risk assessment result, thereby improving accuracy of the spinal health risk assessment result. By assessing the spinal health risk by using the method in this application, the operation is convenient, so that the actual requirement of the user can be quickly and conveniently met, thereby improving user experience of using the electronic device.

### BRIEF DESCRIPTION OF DRAWINGS

FIG. 1 is an example of a diagram of an ATR angle according to an embodiment of this application;
FIG. 2A is a diagram of a hardware structure of an electronic device according to an embodiment of this application;
FIG. 2B is a block diagram of a software structure of an electronic device 100 according to an embodiment of this application;
FIG. 2C is a diagram of a system architecture of an electronic device 100 according to an embodiment of this application;
FIG. 3 shows an example of a user interface that is on an electronic device 100 and that is configured to display installed applications;
FIG. 4A and FIG. 4B show examples of a group of user interfaces related when an electronic device 100 starts an intelligent care application;
FIG. 5A to FIG. 5H show examples of a group of user interfaces related when an electronic device 100 outputs guide information to guide a user;
FIG. 6A to FIG. 6C show examples of another group of user interfaces related when an electronic device 100 outputs guide information to guide a user;
FIG. 7A and FIG. 7B show examples of a group of user interfaces related when an electronic device 100 takes a photo after a user adjusts to a first state;
FIG. 8A and FIG. 8B show examples of a group of user interfaces related when an electronic device 100 records a video after a user adjusts to a first state;
FIG. 9A to FIG. 9F show examples of a group of user interfaces related when an electronic device 100 determines and outputs an assessment result;
FIG. 10 shows an example of a procedure of a spinal health risk assessment method according to an embodiment of this application;
FIG. 11 is an example of a diagram of portrait processing according to an embodiment of this application;
FIG. 12 is an example of a diagram of a first state determining method according to an embodiment of this application;
FIG. 13 is an example of a diagram of another first state determining method according to an embodiment of this application; and
FIG. 14 is an example of a diagram of a feature extraction method according to an embodiment of this application.

### DESCRIPTION OF EMBODIMENTS

The technical solutions in embodiments of this application are clearly described in detail below with reference to the accompanying drawings. In the descriptions of embodiments of this application, unless otherwise stated, "/" represents the meaning of "or". For example, A/B may represent A or B. "And/or" in the text merely describes an association relationship between associated objects and represents that three relationships may exist. For example, A and/or B may represent the following three cases: Only A exists, both A and B exist, and only B exists. In addition, in the descriptions of embodiments of this application, "a plurality of" means two or more than two.

Hereinafter, the terms "first" and "second" are used only for descriptive purposes, and cannot be understood as indicating or implying relative importance or implicitly indicating a quantity of indicated technical features. Therefore, a feature limited by "first" and "second" may explicitly or implicitly include one or more features. In the descriptions of embodiments of this application, unless otherwise stated, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is source code written in a specific computer language such as java or an extensible markup language (extensible markup language, XML). Interface source code is parsed and rendered on an electronic device, and is finally presented as content that can be identified by the user. A frequently used representation form of the user interface is a graphical user interface (graphical user interface, GUI), and is a user interface that is displayed in a graphical manner and that is related to a computer operation. The user interface may be a visual interface element such as a text, an icon, a button, a menu, a tab, a text box, a dialog box, a status bar, a navigation bar, or a widget that is displayed on a display of the electronic device.

Scoliosis is a three-dimensional deformity of a spine, and includes sequence anomalies in coronal, sagittal, and axial views. The spine of a normal person should be a straight line from behind, and two sides of a trunk should be symmetrical. If the spine bends laterally for more than 10 degrees, scoliosis may be diagnosed. Usually, slight scoliosis does not cause an obvious discomfort, and does not cause an obvious body deformity on the appearance. Serious scoliosis affects growth and development of infants and adolescents, causing body deformation. In serious cases, cardiopulmonary functions and even spinal cord may be affected, causing paralysis.

A spinal health risk assessment method may include a forward flexion test method. In the forward flexion test method, a to-be-measured person is located in a place with bright light during the test, and a measurer faces the to-be-measured person with an exposed back. Knees of the to-be-measured person are straight, both feet are put together and are upright, both arms are straight and palms are pressed together. The to-be-measured person slowly bends forward to approximately 90 degrees, and presses both palms together and gradually puts both hands between both knees. Lines of sight of the measurer are parallel to bending of the to-be-measured person. The measurer observes from a cervical vertebra all the way to a waist, and checks whether two sides of the spine are uneven, and whether a unilateral rib has protuberance or whether a unilateral muscle has contracture. Asymmetry that appears at any part of the back may be scoliosis. This method requires participation of professional inspectors, and the measurement result may be affected by a subjective measurement method of the measurer. It is difficult to control an error and the operation is also complex.

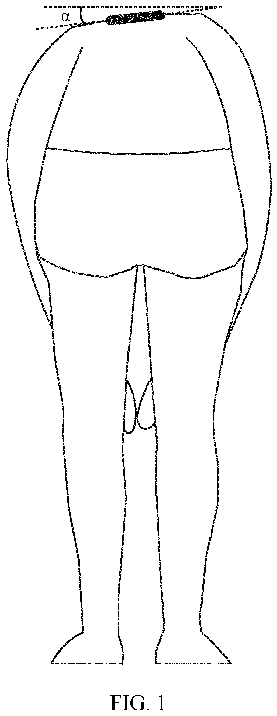

With reference to FIG. 1, an angle of trunk rotation (Angle of Trunk Rotation, ATR) generally indicates severity of scoliosis. During ATR angle measurement, knees of a subject with an exposed back are straight and upright, and both arms are straight and palms are pressed together. The subject lowers a head and slowly bends forward to approximately 90 degrees, and presses both palms together and gradually puts both hands between both knees, to obtain an angle α, namely, the ATR angle between a back tangent of the subject and a horizontal line. A larger ATR corresponds to a higher spinal health risk level.

Embodiments of this application provide a spinal health risk assessment method. The method may be applied to an electronic device including a shooting apparatus and a display apparatus, and the shooting apparatus may include a camera.

In some implementations of this application, the electronic device may guide the user to adjust to a first state, and after determining, by using a preset strategy, that the user is in the first state, start to shoot a photo or record a video, determine a target picture from shot photos or a recorded video, calculate an angle of trunk rotation of the user from the target picture, and map the spinal health risk level based on the angle of trunk rotation, to output a corresponding spinal health risk assessment result, thereby improving accuracy of the spinal health risk assessment result. By assessing the spinal health risk by using the method in this application, convenience and accuracy of the spinal health risk assessment method can be improved, to help optimize use experience of the user.

Spinal health risk assessment may be a function provided by a third-party application, or may be a function provided by a system application of the electronic device. The system application is an application provided or developed by a producer of the electronic device, and the third-party application is an application provided or developed by a producer of a non-electronic device. The producer of the electronic device may include a manufacturer, a supplier, a provider, an operator, or the like of the electronic device. The manufacturer may be a vendor that processes and manufactures the electronic device by making or purchasing parts and raw materials. The supplier may be a vendor that provides a complete device, raw materials, or parts of the electronic device. The operator may be a vendor responsible for distribution of the electronic device.

Herein, for how the electronic device shoots a photo or records a video after determining that the user is in the first state, refer to detailed descriptions in subsequent embodiments. Details are not described herein temporarily.

In some other implementations of this application, the electronic device may obtain a user portrait of a to-be-assessed person by using a front-facing camera, and output a spinal health risk assessment result of the to-be-assessed person in response to a voice control operation of the to-be-assessed person. For detailed content of the spinal health risk assessment result, refer to detailed descriptions of subsequent method embodiments. Details are not described herein temporarily.

In some other implementations of this application, the electronic device may obtain a user portrait of a to-be-assessed person by using a rear-facing camera, and output a spinal health risk assessment result of the to-be-assessed person in response to a user operation of a test assistant. For detailed content of the spinal health risk assessment result, refer to detailed descriptions of subsequent method embodiments. Details are not described herein temporarily.

Before the spinal health risk assessment method is described in detail, the following first describes an electronic device for implementing the method.

A specific type of the electronic device is not limited in embodiments of this application. For example, the electronic device may include a mobile phone, and may further include a tablet computer, a desktop computer, a laptop computer, a handheld computer, a notebook computer, a smart screen, an augmented reality (augmented reality, AR) device, a virtual reality (virtual reality, VR) device, and an artificial intelligence (artificial intelligence, AI) device, and may further include an Internet of Things (Internet of Things, IOT) device or a smart home device such as a smart dressing mirror, a smart television, or a camera. This is not limited thereto. The electronic device may further include a non-portable terminal device such as a laptop (laptop) having a touch-sensitive surface or a touch panel or a desktop computer having a touch-sensitive surface or a touch panel, and the like.

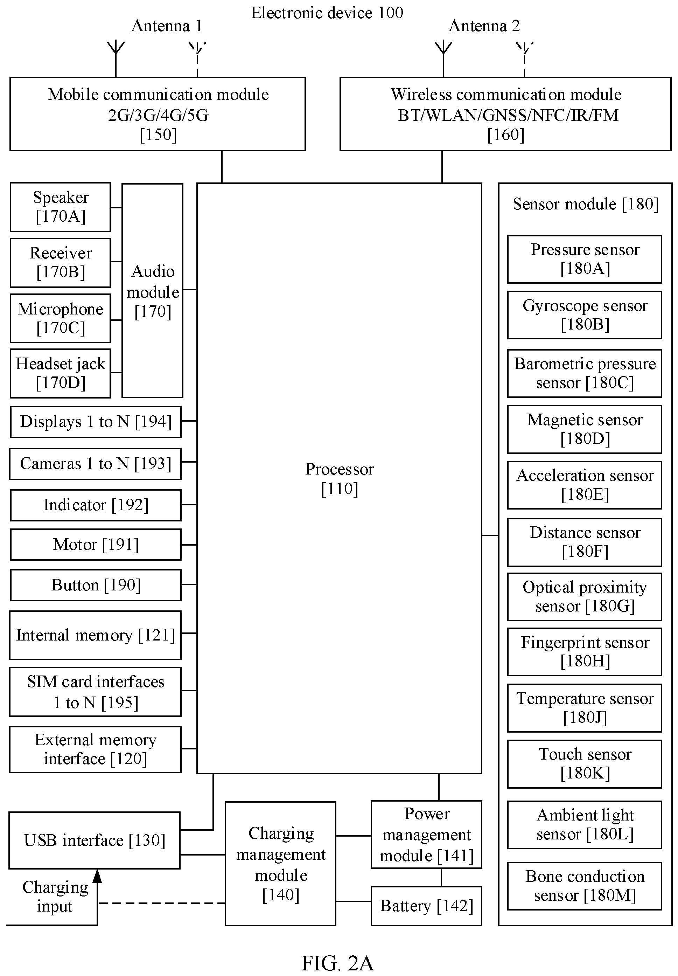

FIG. 2A is a diagram of a hardware structure of an electronic device 100 according to an embodiment of this application.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like.

The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

The structure shown in this embodiment of this application does not constitute a specific limitation on the electronic device 100. In some other implementations of this application, the electronic device 100 may include more or fewer components than those shown in the figure, or combine some components, or split some components, or have different component arrangements. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

In some implementations, the processor 110 such as the controller or the GPU may be configured to: in a spinal health assessment scenario, obtain, in a manner such as filtering, a target photo from a plurality of frames of images captured by the camera 193, and display the target photo in a viewfinder frame.

The controller may be a nerve center and a command center of the electronic device 100. The controller may generate an operation control signal based on instruction operation code and a time sequence signal, to complete control of instruction fetching and instruction execution.

A memory may be further disposed in the processor 110, and is configured to store instructions and data. In some implementations, the memory in the processor 110 is a cache memory. The memory may store an instruction or data that has been used or cyclically used by the processor 110. If the processor 110 needs to use the instruction or the data again, the processor may directly invoke the instruction or the data from the memory. This avoids repeated access, reduces waiting time of the processor 110, and therefore improves system efficiency.

In some implementations, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like. It may be understood that an interface connection relationship between modules that is illustrated in this embodiment of this application is merely an example for description, and does not constitute a limitation on a structure of the electronic device 100. In some other implementations of this application, the electronic device 100 may alternatively use an interface connection manner different from that in the foregoing embodiment, or use a combination of a plurality of interface connection manners.

The charging management module 140 is configured to receive a charging input from a charger. The charger may be a wireless charger or a wired charger. In some embodiments of wired charging, the charging management module 140 may receive a charging input of a wired charger through the USB interface 130. In some embodiments of wireless charging, the charging management module 140 may receive a wireless charging input through a wireless charging coil of the electronic device 100. The charging management module 140 supplies power to the electronic device through the power management module 141 while charging the battery 142.

The power management module 141 is configured to connect to the battery 142, the charging management module 140, and the processor 110. The power management module 141 receives an input of the battery 142 and/or the charging management module 140, to supply power to the processor 110, the internal memory 121, an external memory, the display 194, the camera 193, the wireless communication module 160, and the like. The power management module 141 may be further configured to monitor parameters such as a battery capacity, a battery cycle count, and a battery health status (electric leakage or impedance).

In some other implementations, the power management module 141 may alternatively be disposed in the processor 110. In some other implementations, the power management module 141 and the charging management module 140 may alternatively be disposed in a same device.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication bands. Different antennas may be further multiplexed, to improve antenna utilization. For example, the antenna 1 may be multiplexed as a diversity antenna of a wireless local area network. In some other implementations, the antenna may be used in combination with a tuning switch.

The mobile communication module 150 may provide a wireless communication solution that is applied to the electronic device 100 and that includes 2G/3G/4G/5G, and the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some implementations, at least some functional modules of the mobile communication module 150 may be disposed in the processor 110. In some implementations, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 170A, the receiver 170B, or the like), or displays an image or a video through the display 194. In some implementations, the modem processor may be an independent component. In some other implementations, the modem processor may be independent of the processor 110, and is disposed in a same device as the mobile communication module 150 or another functional module.

The wireless communication module 160 may provide a wireless communication solution that is applied to the electronic device 100 and that includes wireless local area networks (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processing module. The wireless communication module 160 receives an electromagnetic wave through the antenna 2, performs demodulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some implementations, in the electronic device 100, the antenna 1 and the mobile communication module 150 are coupled, and the antenna 2 and the wireless communication module 160 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (Beidou navigation satellite system, BDS), a quasi-zenith satellite system (quasi-zenith satellite system, QZSS), and/or satellite based augmentation systems (satellite based augmentation systems, SBAS).

The external memory interface 120 may be configured to connect to an external storage card, for example, a micro SD card, to extend a storage capability of the electronic device 100. The external storage card communicates with the processor 110 through the external memory interface 120, to implement a data storage function. For example, files such as music and videos are stored in the external storage card.

The internal memory 121 may be configured to store computer-executable program code, and the computer-executable program code includes instructions. The processor 110 runs the instructions stored in the internal memory 121, to perform various function applications and data processing of the electronic device 100. The internal memory 121 may include a program storage area and a data storage area. The program storage area may store an operating system, an application required by at least one function (for example, a sound playing function or an image playing function), and the like. The data storage area may store data (such as audio data and an address book) created during use of the electronic device 100, and the like. In addition, the internal memory 121 may include a high-speed random access memory, or may include a non-volatile memory, for example, at least one magnetic disk storage device, a flash memory device, or a universal flash storage (universal flash storage, UFS).

The electronic device 100 may implement an audio function, for example, music playing and recording, through the audio module 170, the speaker 170A, the receiver 170B, the microphone 170C, the headset jack 170D, the application processor, and the like. The audio module 170 is configured to convert digital audio information into an analog audio signal for output, and is also configured to convert an analog audio input into a digital audio signal. The audio module 170 may be further configured to encode and decode an audio signal. In some implementations, the audio module 170 may be disposed in the processor 110, or some functional modules in the audio module 170 are disposed in the processor 110.

The touch sensor 180K is also referred to as a "touch panel". The touch sensor 180K may be disposed on the display 194, and the touch sensor 180K and the display 194 constitute a touchscreen, which is also referred to as a "touch screen". The touch sensor 180K is configured to detect a touch operation performed on or near the touch sensor. The touch sensor may transmit the detected touch operation to the application processor to determine a type of a touch event. A visual output related to the touch operation may be provided through the display 194. In some other implementations, the touch sensor 180K may alternatively be disposed on a surface of the electronic device 100, at a position different from that of the display 194.

The button 190 includes a power button, a volume button, and the like. The button 190 may be a mechanical button, or may be a touch button. The electronic device 100 may receive a key input, and generate a key signal input related to user settings and function control of the electronic device 100.

The motor 191 may generate a vibration prompt. The motor 191 may be configured to provide an incoming call vibration prompt, or may be configured to provide a touch vibration feedback. For example, touch operations performed on different applications (for example, photographing and audio playing) may correspond to different vibration feedback effects. The motor 191 may also correspond to different vibration feedback effects for touch operations performed on different areas of the display 194. Different application scenarios (for example, an information reminder, an evaluation completion prompt, and a game) may also correspond to different vibration feedback effects. A touch vibration feedback effect may be further customized.

The indicator 192 may be an indicator light, and may be configured to indicate a charging status and a power level change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or more SIM card interfaces. The SIM card interface 195 may support a nano-SIM card, a micro-SIM card, a SIM card, and the like. A plurality of cards may be inserted into a same SIM card interface 195 at the same time. The plurality of cards may be of a same type or different types. The SIM card interface 195 is also compatible with different types of SIM cards. The SIM card interface 195 is also compatible with an external storage card. The electronic device 100 interacts with a network through the SIM card, to implement functions such as call and data communication. In some implementations, the electronic device 100 uses an eSIM, that is, an embedded SIM card. The eSIM card may be embedded into the electronic device 100, and cannot be separated from the electronic device 100.

The camera 193 includes a lens and a photosensitive element (which may also be referred to as an image sensor), configured to capture a still image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP for conversion into a digital image signal, for example, an image signal in a format such as standard RGB or YUV.

Hardware configurations and physical positions of the cameras 193 may be different. Therefore, sizes, ranges, content, definition, or the like of images captured by different cameras may be different.

The electronic device 100 may implement a shooting function through the ISP, the camera 193, the video codec, the GPU, the display 194, the application processor, and the like.

The camera 193 may be disposed on two sides of the electronic device. A camera that is located on a same plane as the display 194 of the electronic device may be referred to as a front-facing camera, and a camera that is located on a plane on which a rear cover of the electronic device is located may be referred to as a rear-facing camera. The front-facing camera may be configured to capture an image of a photographer facing the display 194, and the rear-facing camera may be configured to capture an image of a subject (such as a person or a scenery) facing the photographer.

In some implementations, the camera 193 may be configured to collect depth data. For example, the camera 193 may include a (time of flight, TOF) 3D sensing module or a structured light (structured light) 3D sensing module, and is configured to obtain depth information. A camera configured to collect the depth data may be the front-facing camera or the rear-facing camera.

The video codec is configured to compress or decompress a digital image. The electronic device 100 may support one or more image codecs. In this way, the electronic device 100 may open or store pictures or videos in a plurality of encoding formats.

The electronic device 100 implements a display function through the GPU, the display 194, the application processor, and the like. The GPU is a microprocessor for image processing, and is connected to the display 194 and the application processor. The GPU is configured to perform mathematical and geometric computing for graphics rendering. The processor 110 may include one or more GPUs, which execute program instructions to generate or change display information.

The display 194 is configured to display an image, a video, and the like. The display 194 includes a display panel. The display panel may use a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini LED, a micro LED, a micro-OLED, quantum dot light emitting diodes (quantum dot light emitting diodes, QLED), or the like. In some implementations, the electronic device 100 may include one or N displays 194, where N is a positive integer greater than 1.

The electronic device 100 may implement a shooting function through the ISP, the camera 193, the video codec, the GPU, the display 194, the application processor, and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise and brightness of the image. The ISP may further optimize parameters such as exposure and a color temperature of a shooting scenario. In some implementations, the ISP may be disposed in the camera 193.

The camera 193 is configured to capture a still image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format such as RGB or YUV. In some implementations, the electronic device 100 may include one or N cameras 193, where N is a positive integer greater than 1.

The NPU is a neural-network (neural-network, NN) computing processor, and quickly processes input information with reference to a structure of a biological neural network, for example, a transfer mode between human brain neurons, and may further continuously perform self-learning. Applications such as intelligent cognition of the electronic device 100, for example, image recognition, facial recognition, speech recognition, and text understanding, may be implemented through the NPU.

The internal memory 121 may include one or more random access memories (random access memory, RAM) and one or more non-volatile memories (non-volatile memory, NVM).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a 5th generation DDR SDRAM generally referred to as a DDR5 SDRAM), or the like. The non-volatile memory may include a magnetic disk storage device and a flash memory (flash memory).

According to an operating principle, the flash memory may be classified into a NOR FLASH, a NAND FLASH, a 3D NAND FLASH, or the like; according to a quantity of voltage levels per cell, the flash memory may be classified into a single-level cell (single-level cell, SLC), a multi-level cell (multi-level cell, MLC), a triple-level cell (triple-level cell, TLC), a quad-level cell (quad-level cell, QLC), or the like; and according to storage specifications, the flash memory may be classified into a universal flash storage (English: universal flash storage, UFS), an embedded multi media memory card (embedded multi media Card, eMMC), or the like.

The random access memory may be directly read and written by the processor 110, may be configured to store executable programs (for example, machine instructions) of an operating system or another running program, and may also be configured to store data of a user and an application, and the like.

The non-volatile memory may also store executable programs, data of the user and the application, and the like, and may be loaded into the random access memory in advance, to be directly read and written by the processor 110.

In this embodiment of this application, the processor 110 of the electronic device 100 is configured to: after an intelligent care application is started, detect whether the user adjusts to a first state, and after detecting that the user adjusts to the first state, obtain one or more target photos, perform feature extraction processing on the target pictures, and determine a target ATR angle, where the angle is used to assess a spinal health risk of the user. For a manner in which the processor 110 guides the user to adjust to the first state and a manner in which the processor 110 performs feature extraction processing on the target picture, refer to detailed descriptions of subsequent method embodiments.

In some implementations, the display 194 may be configured to display an image captured by the camera, a first user interface, and guide information. Then, the display 194 may be configured to receive a user operation performed on a camera switching control, a start control, and a shooting/recording switching control. The processor 110 is configured to: in response to the user operation, switch between the front-facing camera and the rear-facing camera, shoot a photo or record a video, and switch to a shooting mode or a recording mode. The photo or the video is obtained through the foregoing operation, and an assessment result is determined and output after analysis. The display 194 may be configured to display the assessment result. For a form in which the display 194 displays the assessment result and related prompt information, refer to detailed descriptions of subsequent method embodiments.

A software system of the electronic device 100 may use a layered architecture, an event-driven architecture, a microkernel architecture, a microservice architecture, or a cloud architecture. In an embodiment of this application, an Android system with a layered architecture is used as an example to describe a software structure of the electronic device 100.

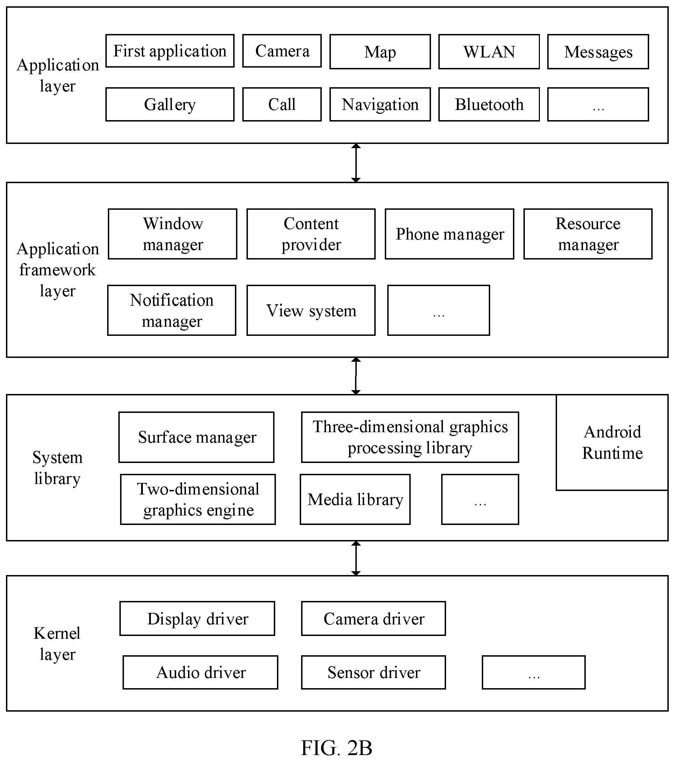

FIG. 2B is a block diagram of a software structure of an electronic device 100 according to an embodiment of this application.

In the layered architecture, software is divided into several layers, and each layer has a clear role and task. The layers communicate with each other through a software interface. In some implementations, the Android system is divided into four layers: an application layer, an application framework layer, an Android Runtime (Android Runtime) and a system library, and a kernel layer from top to bottom.

The application layer may include a series of application packages.

As shown in FIG. 2B, the application package may include applications such as a first application, a camera application, Gallery, Call, Map, Navigation, WLAN, Bluetooth, Music, Video, and Messages.

In this embodiment of this application, the first application may be a system application, or may be a third-party application. The first application may support the electronic device 100 in starting physical health assessment when obtaining touch of the user, and the user may obtain spinal health risk assessment based on physical health assessment. The first application may be an intelligent care application.

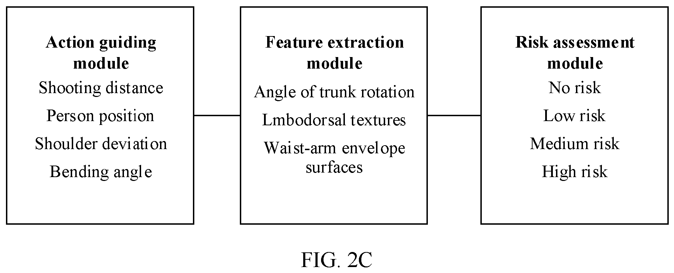

As shown in FIG. 2C, a system architecture of the first application may include an action guiding module, a feature extraction module, and a risk assessment module. The action guiding module is configured to guide a shooting distance, a person position, a shoulder deviation, a bending angle, and the like of the user. The feature extraction module is configured to extract an angle of trunk rotation, lumbodorsal textures, waist-arm envelope surfaces, and the like. The risk assessment module is configured to assess a spinal health risk level, where the spinal health risk level may be none, low, medium, or high.

The camera application is configured to provide a photo and video shooting function, and may be a system application or a third-party application.

The application framework layer provides an application programming interface (application programming interface, API) and a programming framework for an application at the application layer. The application framework layer includes some predefined functions.

As shown in FIG. 2B, the application framework layer may include a window manager, a content provider, a view system, a phone manager, a resource manager, a notification manager, and the like.

The window manager is configured to manage a window program. The window manager may obtain a size of the display, determine whether there is a status bar, lock the screen, take a screenshot, and the like.

The content provider is configured to: store and obtain data, and enable the data to be accessed by an application. The data may include a video, an image, audio, calls made and answered, a browse history and a bookmark, a personal address book, and the like.

The view system includes a visual control, for example, a text display control or a picture display control. The view system may be configured to construct an application. A display interface may include one or more views. For example, a display interface including a message notification icon may include a text display view and a picture display view.

The phone manager is configured to provide a communication function for the electronic device 100, for example, call status management (including connection, hang-up, and the like).

The resource manager provides various resources for an application, such as a localized string, an icon, a picture, a layout file, and a video file.

The notification manager enables an application to display, in the status bar, notification information, which may be used for conveying a notification-type message that may automatically disappear after a short stay without user interaction. For example, the notification manager is configured to: notify download completion, provide a message prompt, and the like. The notification manager may alternatively be a notification that appears in a top status bar of the system in a form of a chart or scroll bar text, for example, a notification of an application running on the background or a notification that appears on the screen in a form of a dialog window. For example, text information is prompted in the status bar, a prompt tone is given, the electronic device vibrates, or an indicator light flashes.

The Android Runtime includes a core library and a virtual machine. The Android Runtime is responsible for scheduling and management of the Android system.

The core library includes two parts: a function that needs to be called in java language and a core library of Android.

The application layer and the application framework layer run in the virtual machine. The virtual machine executes java files at the application layer and the application framework layer as binary files. The virtual machine is configured to execute functions such as object life cycle management, stack management, thread management, security and exception management, and garbage collection.

The system library may include a plurality of functional modules, for example, a surface manager (surface manager), media libraries (Media Libraries), a three-dimensional graphics processing library (for example, an OpenGL ES), a 2D graphics engine (for example, an SGL), and the like.

The surface manager is configured to: manage a display subsystem and provide fusion of 2D and 3D layers for a plurality of applications.

The media library supports playback and recording of a plurality of common audio and video formats, still image files, and the like. The media library may support a plurality of audio and video encoding formats, for example, MPEG 4, H.264, MP3, AAC, AMR, JPG, and PNG.

The three-dimensional graphics processing library is configured to implement three-dimensional graphics drawing, image rendering and synthesis, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The structure shown in this embodiment of this application does not constitute a specific limitation on the electronic device 100. In some other implementations of this application, the electronic device 100 may include more or fewer modules than those shown in the figure.

The following describes a group of user interfaces according to an embodiment of this application.

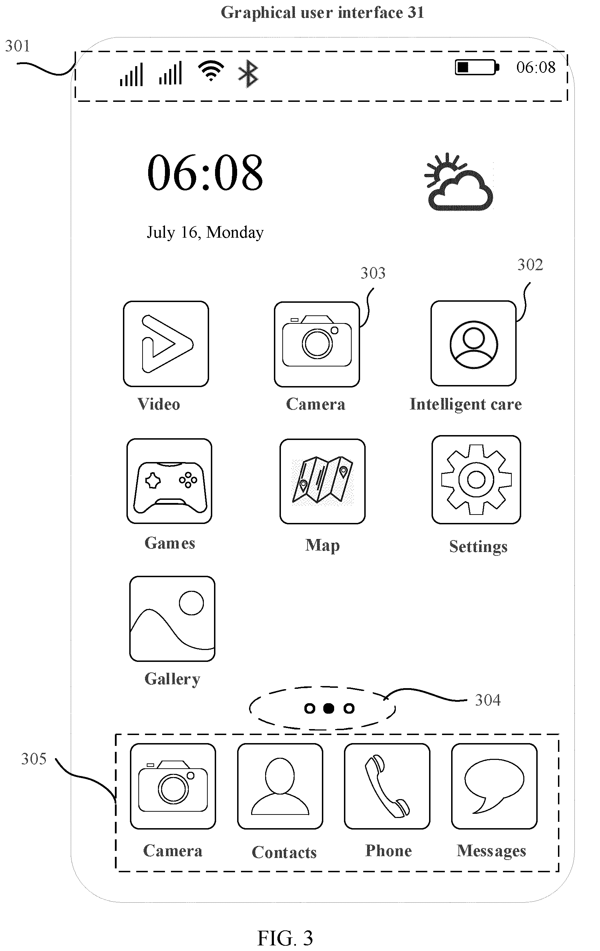

FIG. 3 shows an example of a user interface 31 that is on the electronic device 100 and that is configured to display installed applications.

The user interface 31 displays: a status bar 301, an intelligent care application 302, a camera application 303, a page indicator 304, a tray 305 having icons of frequently used applications, and other application icons.

The status bar 301 may include one or more signal strength indicators of a mobile communication signal (which may also be referred to as a cellular signal), a Bluetooth indicator, one or more signal strength indicators of a Wi-Fi signal, a battery status indicator, a time indicator, and the like.

The intelligent care application 302 is an APP that is installed in the electronic device, configured to assess a spinal health risk, and configured to output an assessment result of the spinal health risk. The intelligent care application may recommend different types of sports games based on different spinal health risk levels. The intelligent care application may be a system application, or may be a third-party application.

The camera application 303 is an APP that is installed in the electronic device, configured to shoot an image or record a video, and configured to capture a picture and a video of a user when the intelligent care application needs to obtain a user image. A spinal health risk assessment function may be integrated into the camera application.

The page indicator 304 may indicate a specific page on which the user is currently browsing an application icon. In this embodiment of this application, application icons may be distributed on a plurality of pages, and the user may flick left or right to browse the application icons on different pages.

The tray 305 having icons of frequently used applications may display: a Phone icon, a Messages icon, a Camera icon, a Contacts icon, and the like.

The other application icons may include, for example, an icon of a video application, an icon of a game application, an icon of a setting application, and an icon of a gallery application.

The game application is an application installed in the electronic device and configured to provide a game downloading and sharing function. A game recommended by the intelligent care application may be opened through the game application.

The video application is a network video application.

The network video application is configured to provide functions such as online watching and downloading of a network video. The network video application may be used to watch a video of a rehabilitation action suggested by the intelligent care application. The network video application may be a system application or a third-party application.

The gallery application may be configured to display a picture and a video shot by the intelligent care application, and may be further configured to display pictures and videos shot by other applications.

This is not limited thereto. More applications may be further installed in the electronic device 100, and icons of these applications may be displayed on a display. For example, a booking application or the like may be further installed in the electronic device 100. The booking application may be configured to make an appointment for a medical service when a spinal health risk level is high.

Names of the foregoing applications are merely words used in embodiments of this application, meanings represented by the names have been recorded in the embodiments, and the names of the applications cannot constitute any limitation on the embodiments. For example, the intelligent care application may also be referred to as other nouns, such as physical health assessment, spinal health risk assessment, and the like.

This is not limited thereto. The user interface 31 shown in FIG. 3 may further include a navigation bar, a sidebar, and the like. In some other implementations, the user interface 31 shown in FIG. 3 as an example may be referred to as a home screen (home screen).

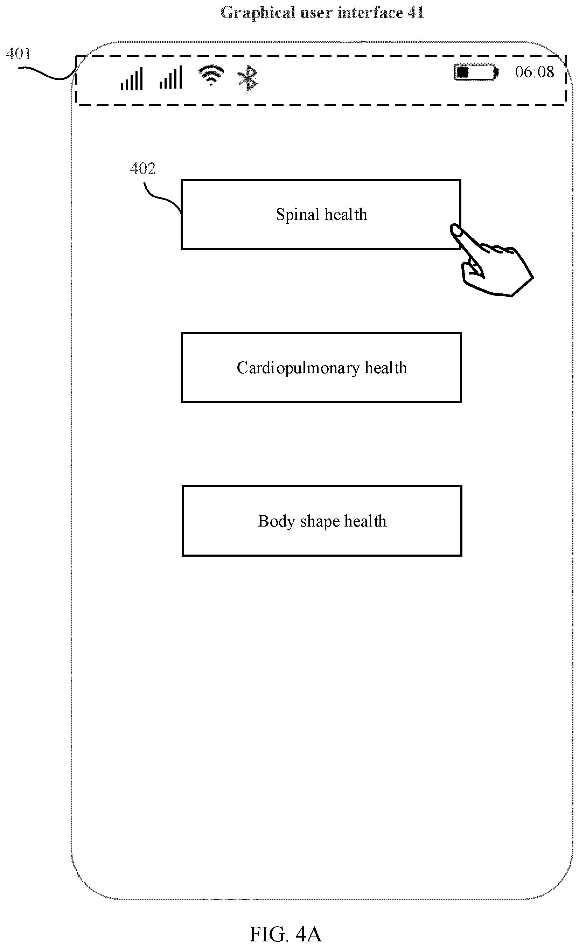

FIG. 4A and FIG. 4B, FIG. 5A and FIG. 5B, and FIG. 6A and FIG. 6B show examples of user interfaces provided by the electronic device 100 when the electronic device 100 assesses a spinal health risk in several scenarios.

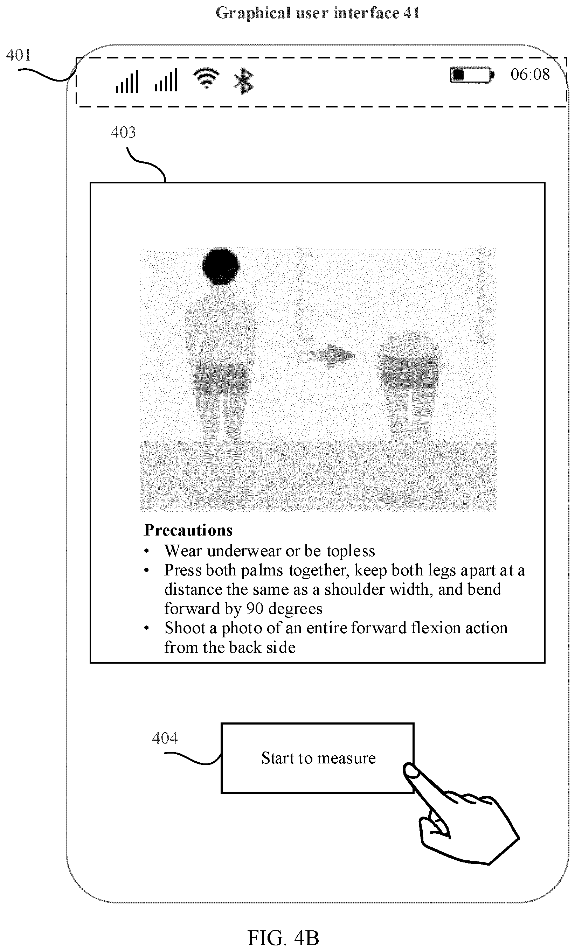

FIG. 4A and FIG. 4B show examples of a group of user interfaces related when an electronic device 100 starts an intelligent care application.

FIG. 4A shows an example of a user interface 41 provided by an intelligent care application after the electronic device 100 starts the intelligent care application. The user interface 41 may be an interface displayed by the electronic device 100 in response to tapping, by the user, the icon 302 of the intelligent care application.

As shown in FIG. 4A, the user interface 41 displays a status bar 401, a spinal health assessment control 402, a cardiopulmonary health assessment control, a body shape health assessment control, and the like.

In some implementations, the spinal health assessment control 402 is configured to provide the user with a function of starting assessment of a spinal health risk. The electronic device 100 may start to assess the spinal health risk in response to a user operation received by the spinal health assessment control 402, and display a user interface shown in FIG. 4B.

This is not limited to the user operation performed on the spinal health assessment control 402 shown in FIG. 4A. In some other implementations of this application, the electronic device 100 may alternatively start "spinal health risk assessment" in another manner.

For example, the electronic device 100 may further jump to an intelligent assessment user interface in response to touching, by the user, the icon of the intelligent care application. The intelligent assessment user interface may include a physical health assessment control. The electronic device 100 displays a user interface shown in FIG. 4A in response to receiving touching, by the user, the physical health assessment control. Alternatively, the electronic device 100 may further start "spinal health risk assessment" and the like in response to a voice instruction received after the user starts the intelligent care application.

As shown in FIG. 4B, after the electronic device 100 responds to touching, by the user, the spinal health assessment control 402, the user interface 41 displays a measurement guide 403, a measurement start control 404, and the like.

The measurement guide 403 may include a standard state diagram, precautions, and the like to be notified to the user. A standard state includes a standard position and a standard posture. The standard position means that a portrait of the user is located in a standard guiding block diagram. The standard posture means that both legs keep apart at a distance the same as a shoulder width, a body is bent forward by 90 degrees, a back side faces the camera, or both palms are pressed together and center on both knees. The precautions include that the user should wear underwear or is topless during measurement, and that a photo of an entire forward flexion action is shot from the back side, and the like. By reading the measurement guide, the user can learn about the precautions required for spinal health assessment, the action posture to be completed, and the like.

In some implementations, the electronic device 100 starts, in response to touching, by the user, the measurement start control 404, the camera to capture an image, and outputs guide information based on a portrait in the captured image, to guide the user to adjust to a first state. The first state includes a first position and a first posture. A distance between the first position and the standard position is less than a first value, and a difference between the first posture and the standard posture is less than a second value. In this way, the spinal health risk of the user can be assessed.

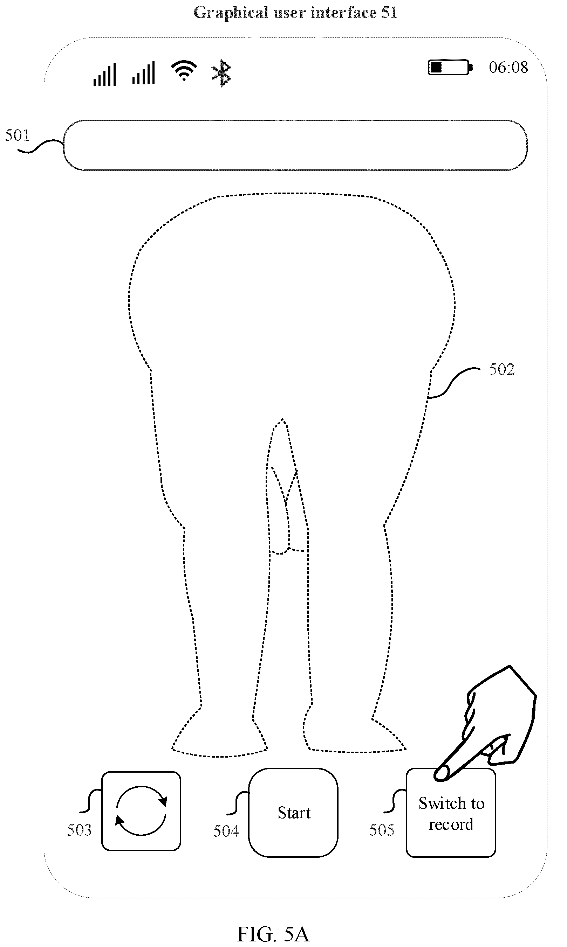

FIG. 5A to FIG. 5H show examples of a group of user interfaces related when an electronic device 100 outputs guide information to guide a user.

FIG. 5A shows an example of a group of user interfaces 51 related when the electronic device 100 guides the user. The user interface 51 may be an interface displayed by the electronic device 100 in response to touching, by the user, the measurement start control 404.

As shown in FIG. 5A, the user interface 51 displays a status bar, an information prompt box 501, a standard guide block diagram 502, a bottom menu bar, and the like.

The bottom menu bar may include a camera switching control 503, a start control 504, a shooting/recording switching control 505, and the like.

The camera switching control 503 is configured to provide a function of switching between a front-facing camera and a rear-facing camera. For example, after receiving an operation of touching, by the user, the camera switching control 503, the electronic device 100 obtains a currently used first camera, and switches the first camera to a second camera. The first camera may be the front-facing camera, and the second camera may be the rear-facing camera.

The start control 504 is configured to: after the electronic device 100 receives that the user is in the first state, prompt the user to start to shoot a photo or record a video, where the first state includes the first position and the first posture, the distance between the first position and the standard position is less than the first value, and the difference between the first posture and the standard posture is less than the second value.

In some implementations, when the electronic device 100 does not receive that the user is in the first state, the start control is displayed in red, and when the electronic device 100 receives that the user is in the first state, the start control is displayed in green.

This is not limited to the method for prompting, by using the start control, the user to start to shoot a photo or record a video. In some other implementations, when detecting that the user is in the first state, the electronic device 100 prompts, by using voice information, the user to start to shoot a photo or record a video.

In some implementations, the electronic device 100 starts to shoot a photo or record a video in response to an operation of touching, by the user, the start control 504, or in response to voice control of the user. In this case, the electronic device 100 may prompt, by using voice information, the user that "a photo is being shot" or "a video is being recorded", or may prompt the user by using an information prompt box. This is not limited herein.

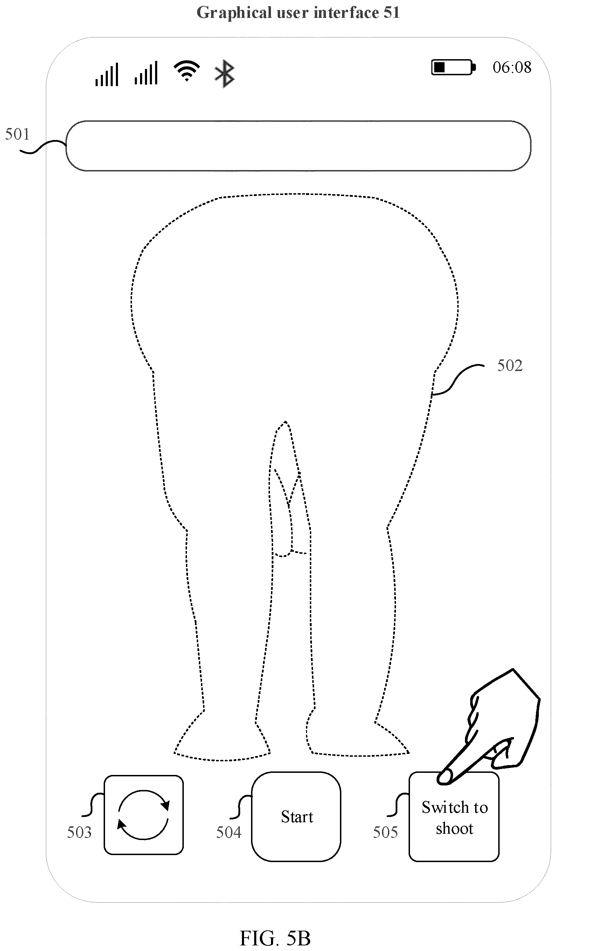

The shooting/recording switching control 505 is configured to provide a function of switching between a photo shooting mode and a video recording mode. With reference to FIG. 5A and FIG. 5B, when the electronic device 100 is in the photo shooting mode, the shooting/recording switching control 505 is displayed as "Switch to record". In this case, the electronic device 100 may switch to the video recording mode in response to a user operation. When the electronic device 100 is in the video recording mode, the shooting/recording switching control 505 is displayed as "Switch to shoot". In this case, the electronic device 100 may switch to the photo shooting mode response to a user operation.

In some implementations, the electronic device 100 displays a user interface shown in FIG. 5A in response to an operation of touching, by the user, the shooting/recording switching control 505. The electronic device 100 performs video recording after detecting that the user is in the first state and receiving that the user touches the start control.

In some other implementations, the electronic device 100 guides the user in response to touching, by the user, the measurement start control 404, to assess a spinal health risk level of the user after the user is in the first state. A user interface shown in FIG. 5B is shown.

As shown in FIG. 5B, the user interface 51 displays the status bar, the information prompt box 501, the standard guide block diagram 502, the bottom menu bar, and the like.

The bottom menu bar may include the camera switching control 503, the start control 504, the shooting/recording switching control 505, and the like.

The electronic device 100 displays the user interface shown in FIG. 5A in response to the operation of touching, by the user, the shooting/recording switching control 505.

In some implementations, the electronic device 100 captures a portrait of the user by invoking a shooting apparatus, and guides the user by using the information prompt box 501 and the standard guide block diagram 502. Specifically, the electronic device recognizes positions of body nodes of the user in real time by using a bone node algorithm, and estimates a portrait position, a body forward flexion angle, and a shoulder deviation based on a proportional relationship and a position relationship between the nodes, to perform corresponding guiding on a position and a posture of the user.

The standard guide block diagram 502 may be dynamic or static. The dynamic standard guide block diagram means that a height and a width of the standard guide block diagram are properly adjusted based on data such as a height and a weight of a user, and the static standard guide block diagram means that the standard guide block diagram is displayed in a preset preview area. The electronic device 100 may detect whether the portrait of the user is located inside the standard guide block diagram 502, whether a size of the portrait of the user is moderate, and whether the user is in the first state.

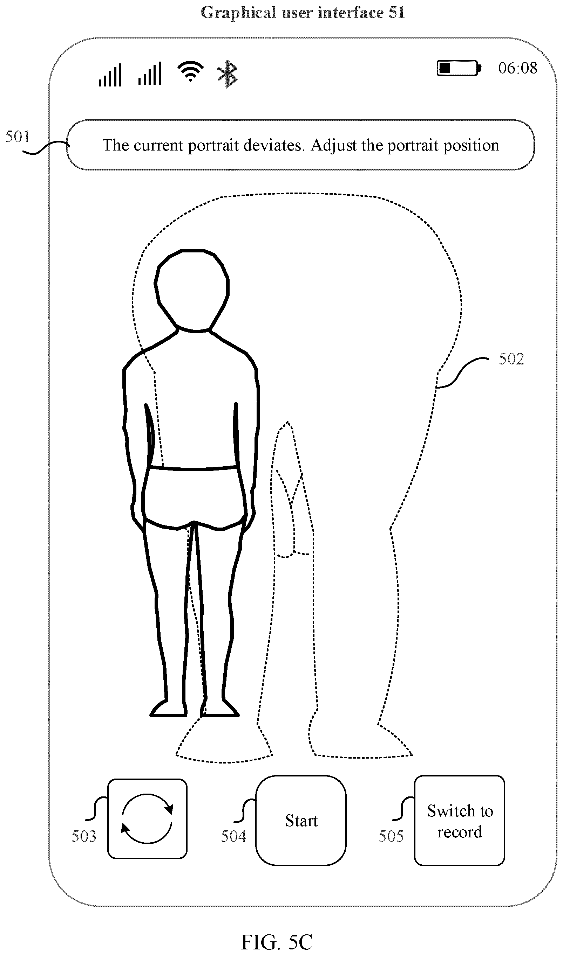

In some implementations, as shown in FIG. 5C, when detecting that the portrait of the user deviates from the standard guide block diagram 502, the electronic device 100 reminds and guides the user by using voice information and/or text information in the information prompt box 501, so that the user adjusts a portrait position of the user to the first position.

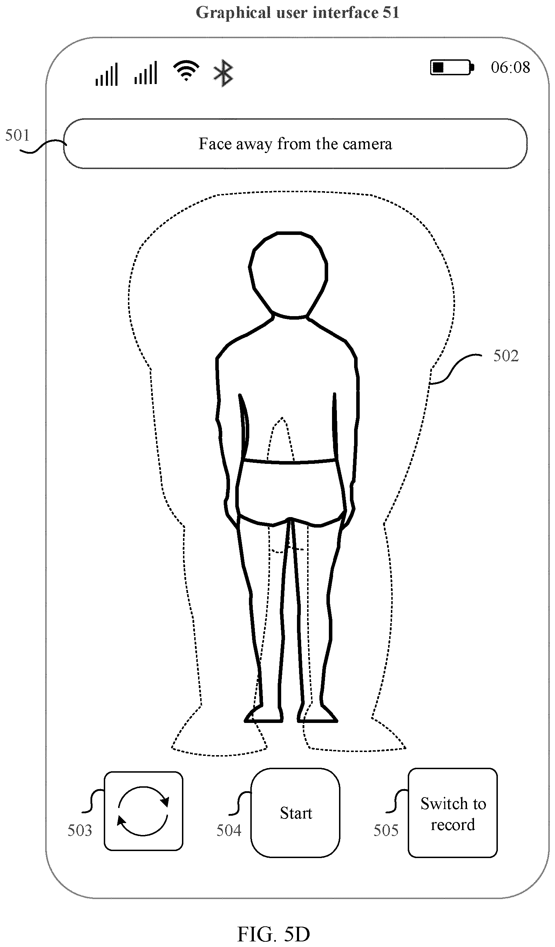

The electronic device 100 recognizes body node positions of the user in the portrait in real time by using the bone node algorithm. When detecting that a front side of the user faces the electronic device 100, the electronic device 100 guides, by using voice information and/or the information prompt box 501, the user to face the electronic device 100 with a back side, and displays a user interface shown in FIG. 5D.

The electronic device 100 recognizes the body node positions of the user in the portrait in real time by using the bone node algorithm. After detecting that the back side of the user faces the electronic device 100, the electronic device 100 determines, by using the position relationship between the body nodes of the user, whether both legs of the user keep apart at a distance the same as a shoulder width, and detects, by using the proportional relationship between the body nodes of the user, whether the body of the user is bent forward by 90 degrees, whether both legs keep apart at a distance the same as a shoulder width, and whether both palms of the user are pressed together and center on both knees.

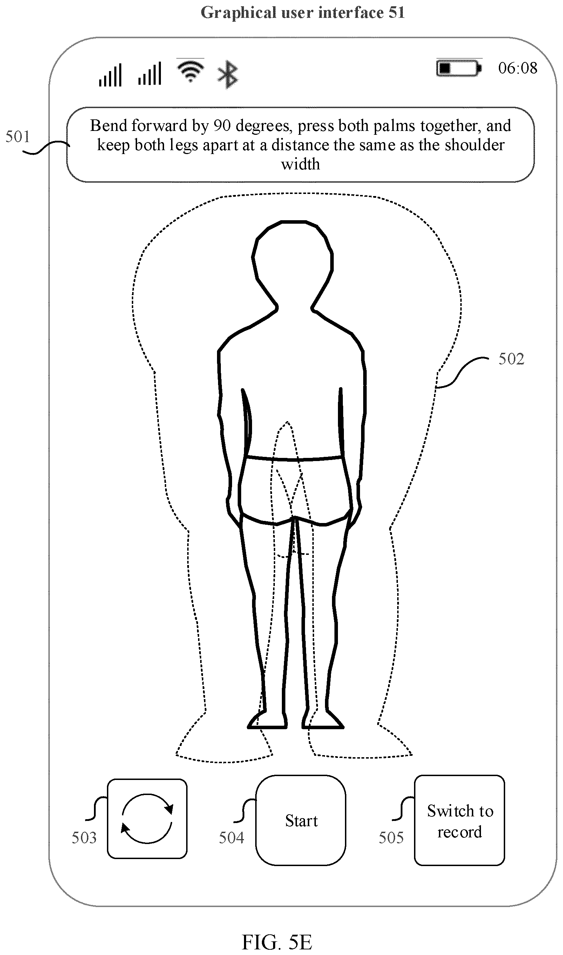

If the electronic device 100 detects that the user is not in a state in which the body is bent forward by 90 degrees, and/or that the user is not in a state in which both palms are pressed together and center on both knees, the electronic device 100 guides, by using voice information and/or the information prompt box 501, the user to adjust a hand posture to the first posture, and displays a user interface shown in FIG. 5E.

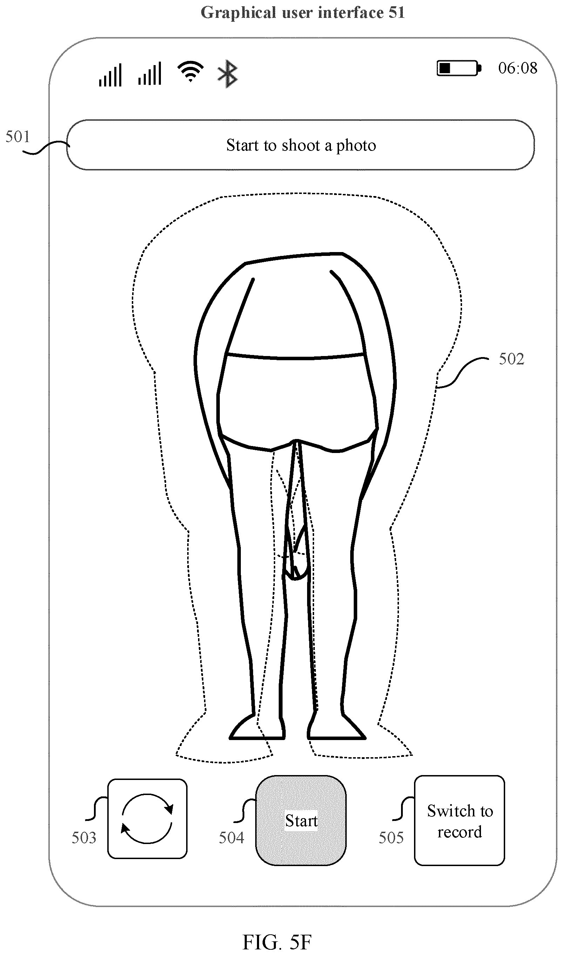

When the electronic device 100 detects that the user is in the first state, the start control is displayed in green, and the electronic device 100 may prompt, by using the start control, the user to start to shoot a photo, or the electronic device 100 may prompt, by using voice information and/or the information prompt box, the user to start to shoot a photo, as shown in a user interface in FIG. 5F.

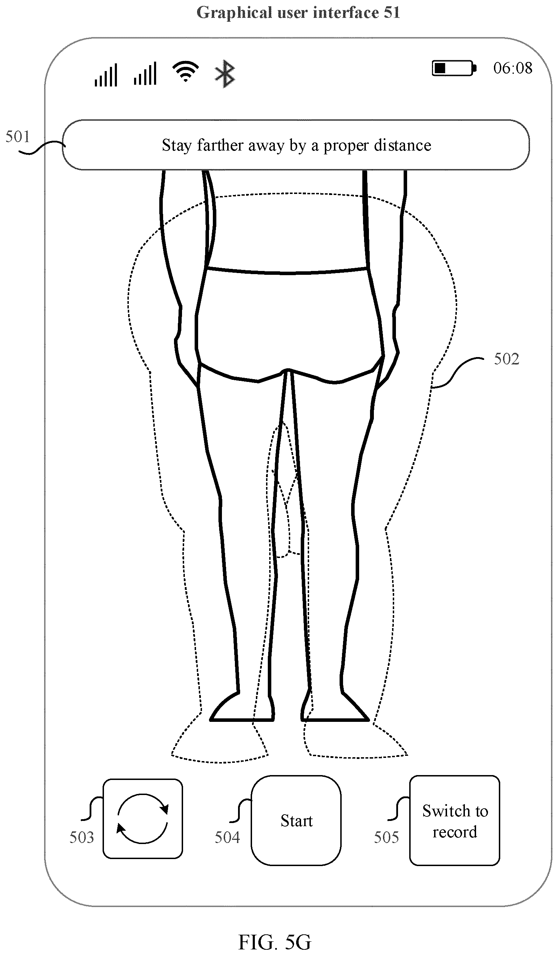

In some other implementations, as shown in FIG. 5G, when detecting that the portrait of the user is not completely displayed or is excessively large, the electronic device 100 guides, by using voice information and/or the information prompt box 501, the user to stay away from the electronic device 100 until the portrait of the user is completely and clearly displayed.

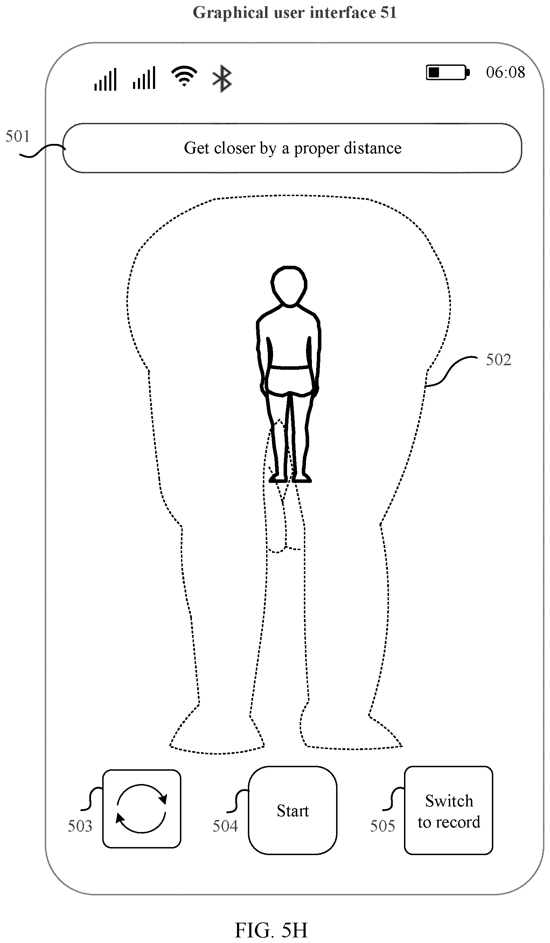

In some other implementations, as shown in FIG. 5H, when detecting that the portrait of the user is not displayed clearly or the portrait is excessively small, the electronic device 100 guides, by using voice information and/or the information prompt box 501, the user to approach the electronic device 100, or re-adjusts a focal length until the portrait of the user is displayed completely and clearly.

This is not limited to guiding the user to approach the electronic device 100, as shown in FIG. 5H. In some other implementations of this application, the electronic device 100 may alternatively adjust the portrait of the user by performing proportional scaling processing on the size of the portrait.

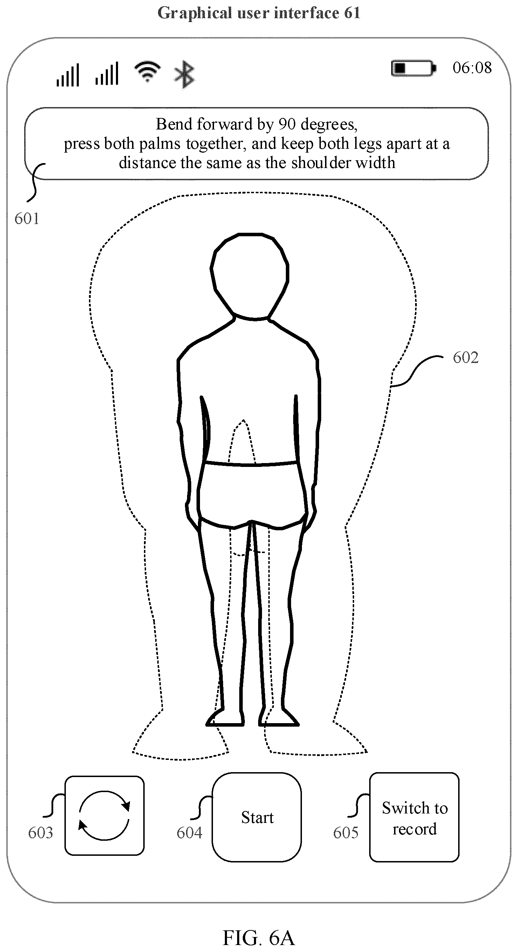

FIG. 6A to FIG. 6C show examples of another group of user interfaces related when an electronic device 100 outputs guide information to guide a user.

FIG. 6A shows an example of a group of user interfaces 61 related when the electronic device 100 guides the user. The user interface 61 may be an interface displayed by the electronic device 100 in response to touching, by the user, the measurement start control 404.

As shown in FIG. 6A, the user interface 61 displays a status bar, an information prompt box 601, a standard guide block diagram 602, a bottom menu bar, and the like.

The bottom menu bar may include a camera switching control 603, a start control 604, a shooting/recording switching control 605, and the like.

In some implementations, the electronic device 100 obtains the portrait of the user by invoking the shooting apparatus, and guides the user by using the information prompt box 601 and the standard guide block diagram 602. Specifically, the electronic device recognizes the positions of the body nodes of the user in real time by using the bone node algorithm, and estimates the portrait position, the body forward flexion angle, and the shoulder deviation based on the proportional relationship and the position relationship between the nodes, to perform corresponding guiding on the user.

The standard guide block diagram 602 may be dynamic or static. The electronic device 100 may detect whether the portrait of the user is located inside the standard guide block diagram 602, whether the size of the portrait of the user is moderate, and whether the user is in the first posture. The first posture is a posture in which a difference between the posture of the user and the standard posture is less than the second value.

In some implementations, the electronic device 100 recognizes the body node positions of the user in the portrait in real time by using the bone node algorithm, determines, by using the position relationship between the body nodes of the user, whether both legs of the user keep apart at a distance the same as a shoulder width, and detects, by using the proportional relationship between the body nodes of the user, whether the body of the user is bent forward by 90 degrees, whether both legs keep apart at a distance the same as a shoulder width, and whether both palms of the user are pressed together and center on both knees. If the electronic device 100 detects that the body of the user is not bent forward by 90 degrees and/or both palms are not in a state of being pressed together and centered on both knees, the electronic device 100 guides, by using voice information and/or the information prompt box 501, the user to adjust the hand posture to the first posture, and displays a user interface shown in FIG. 6A.

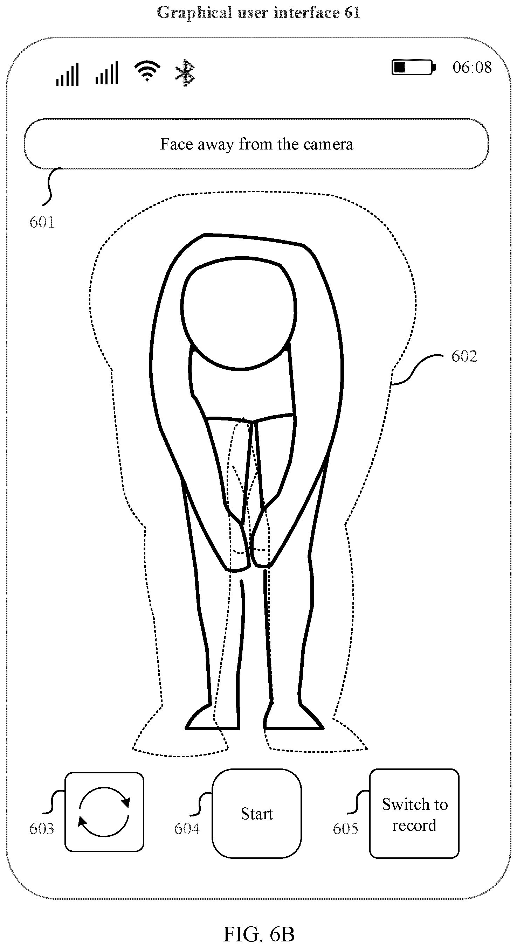

When detecting that the front side of the user faces the electronic device 100, the electronic device 100 guides, by using voice information and/or the information prompt box 501, the user to face the electronic device 100 with the back side, and displays a user interface shown in FIG. 6B.

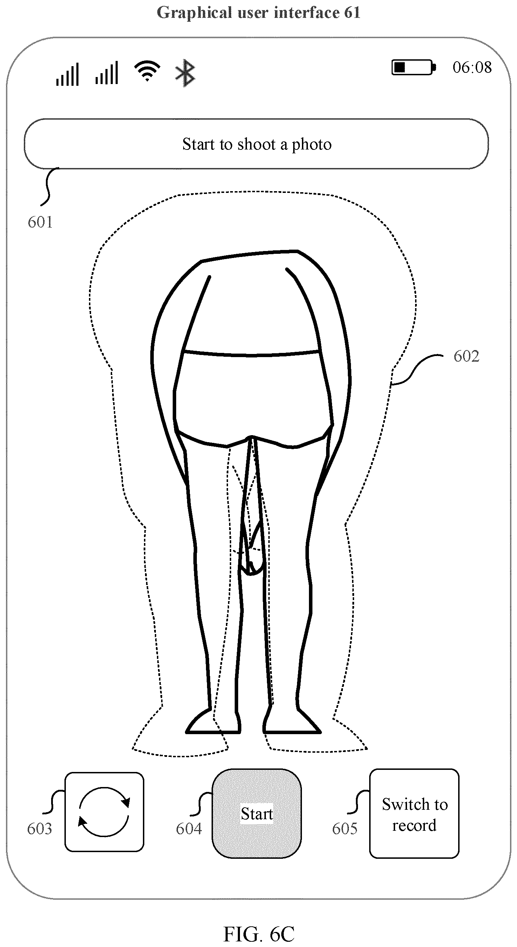

When the electronic device 100 detects that the user is in the first state, the start control is displayed in green, and the electronic device 100 may prompt, by using the start control, the user to start to shoot a photo, or the electronic device 100 may prompt, by using voice information, the user to start to shoot a photo, as shown in a user interface in FIG. 6C.

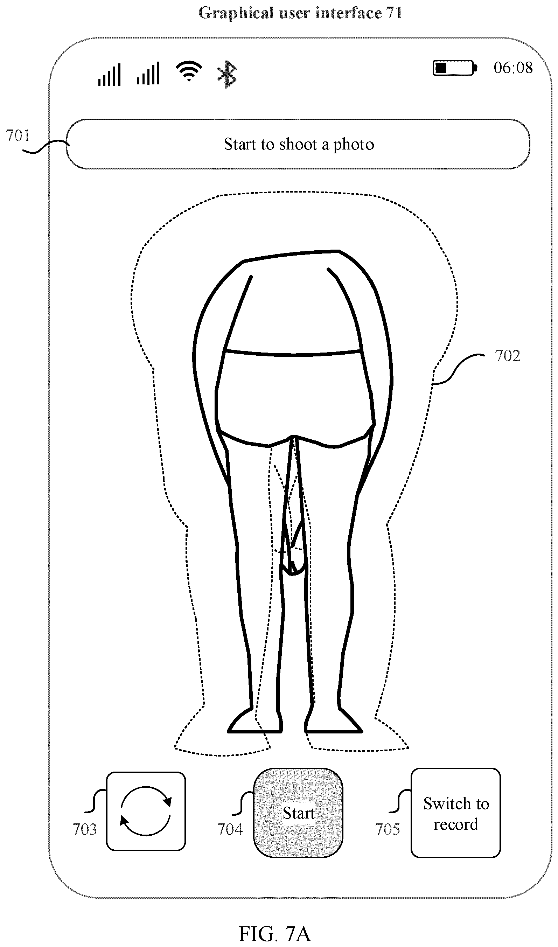

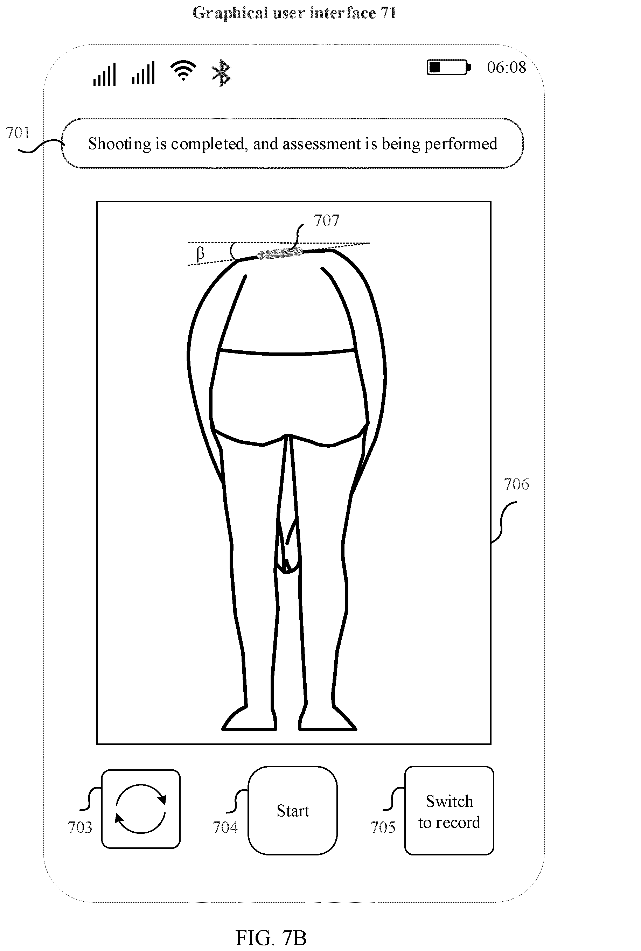

FIG. 7A and FIG. 7B show examples of a group of user interfaces related when an electronic device 100 takes a photo after a user adjusts to a first state.

FIG. 7A shows an example of a user interface 71 related when an electronic device 100 obtains a target picture after a user adjusts to a first state.

As shown in FIG. 7A, the user interface 71 displays a status bar, an information prompt box 701, a standard guide block diagram 702, a bottom menu bar, and the like.

The bottom menu bar may include a camera switching control 703, a start control 704, a shooting/recording switching control 705, and the like.

In some implementations, when detecting that the user is in the first state, the electronic device 100 determines that guiding is completed, and the start control 704 is displayed in green. In response to a user operation of touching the start control 704, the electronic device 100 starts to shoot a real-time photo of the user, generates a target picture 706, and displays a user interface shown in FIG. 7B.

The information prompt box 701 is used to notify the user of a photo shooting progress and progress information of spinal health risk assessment. The electronic device 100 may alternatively notify the user of the photo shooting progress and the progress information of spinal health risk assessment by using voice information.

The target picture 706 may be one or more photos in which the back side of the user faces the electronic device 100.

As shown in FIG. 7B, the electronic device 100 may perform portrait segmentation processing on the target picture 706, that is, extract a first portrait from the target picture 706 by using a deep neural network UNET, then perform edge extraction on the first portrait, to extract edge feature points of the portrait, then determine a human body midline position in the first portrait, that is, estimate the human body midline position based on left and right side positions on an edge of the portrait, finally search for a tangent, determine, from the edge feature points of the first portrait, a first tangent point and a second tangent point that are respectively located on a left side and a right side of the human body midline position, and determine a back tangent 707 including the first tangent point and the second tangent point, where the back tangent 707 is used to calculate a target ATR angle β, and the target ATR is an angle between the back tangent and a horizontal line.

Optionally, a process in which the electronic device 100 performs portrait segmentation processing on the target picture 706 may be displayed on the user interface, or may not be displayed on the user interface. This is not limited herein.

Optionally, after detecting that the user is in the first state, the electronic device 100 may start the camera application to shoot an image, and generate the target picture.

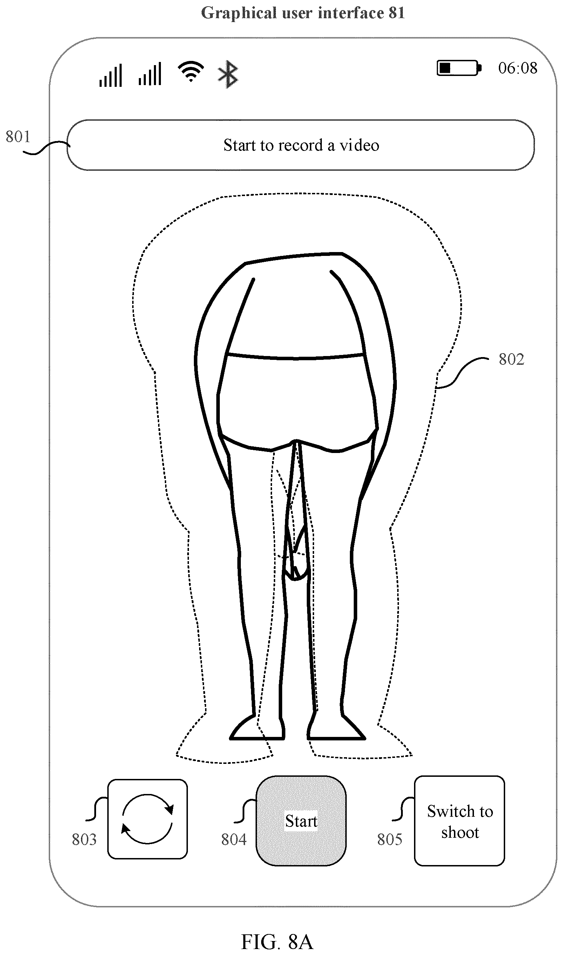

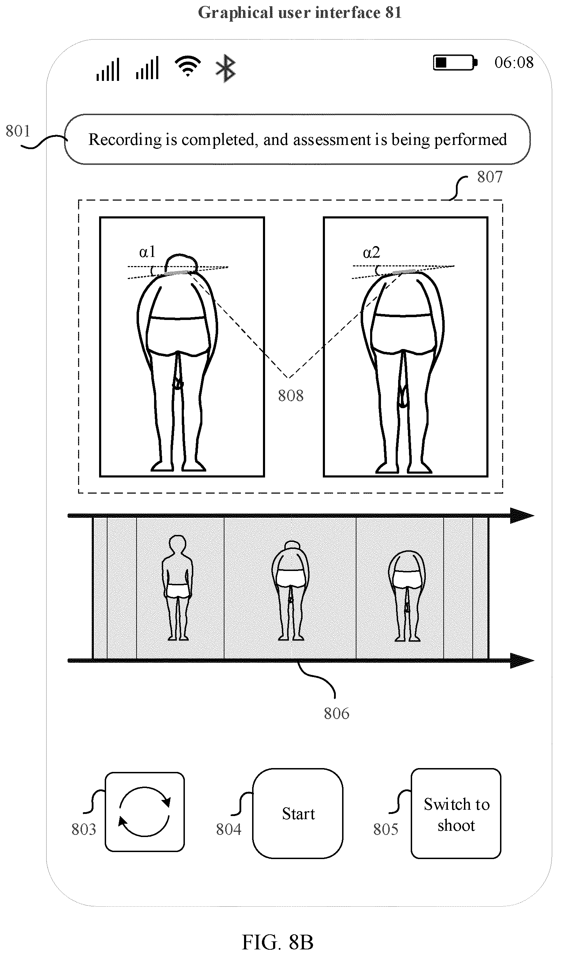

FIG. 8A and FIG. 8B show examples of a group of user interfaces related when an electronic device 100 records a video after a user adjusts to a first state.

As shown in FIG. 8A, a user interface 81 displays a status bar, an information prompt box 801, a standard guide block diagram 802, a bottom menu bar, and the like.

The bottom menu bar may include a camera switching control 803, a start control 804, a shooting/recording switching control 805, and the like.

In some implementations, as shown in FIG. 8A, when detecting that the user is in the first state, the electronic device 100 determines that guiding is completed, and prompts, by using a voice prompt and/or the information prompt box 801, to record a back side video in which the user stands and then bends forwards for 90°.

In some implementations, the electronic device 100 starts to record a target video 806 in response to a user operation of touching the start control 804. After recording is completed, the start control 804 is displayed in green. The electronic device 100 starts to record a video in response to the user operation of touching the start control 804, and prompts, by using the information prompt box 801, that recording is completed. The electronic device 100 extracts key frames from the recorded video, generates a picture set, and performs filtering on the picture set based on body forward flexion angles to extract a plurality of target pictures 807, determines a back tangent 808 in each of the plurality of target pictures, calculates a plurality of rotation angles such as α1 and α2, selects a largest rotation angle of the plurality of rotation angles as the target ATR angle, and displays a user interface shown in FIG. 8B.

Optionally, after completing guiding, the electronic device 100 may start the camera application to record a video, where the video is used by the intelligent care application to generate the target picture.

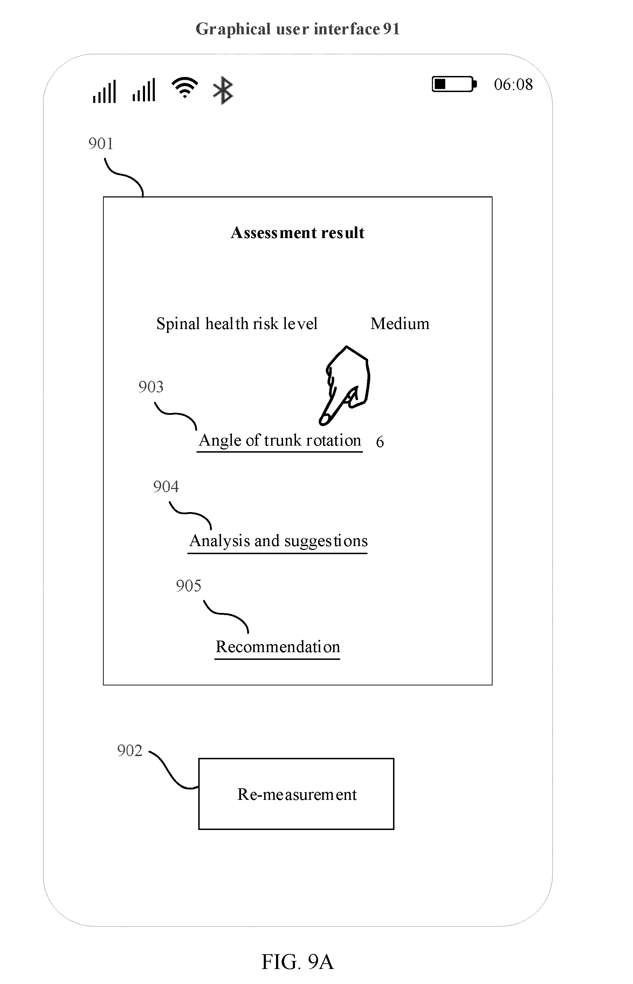

FIG. 9A to FIG. 9F show examples of a group of user interfaces related when an electronic device 100 determines and outputs an assessment result.

FIG. 9A shows an example of a user interface 91 related when an electronic device 100 displays a spinal health risk assessment result.

As shown in FIG. 9A, the user interface 91 displays a status bar, an assessment result display area 901, a re-measurement control 902, and the like.

The assessment result display area 901 includes a spinal health risk level, which may be low, medium, high, or the like.

The assessment result display area 901 may include an angle of trunk rotation control 903, an analysis and suggestions control 904, a recommendation control 905, and the like.

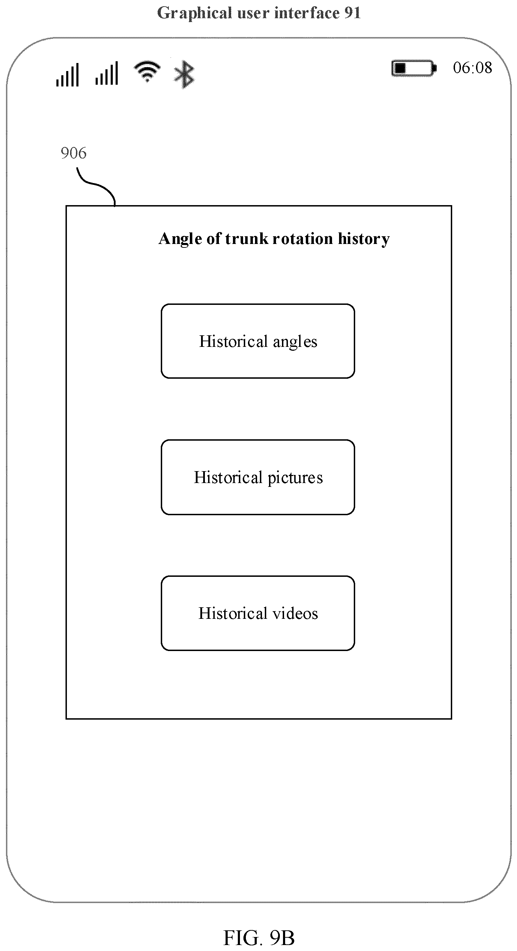

The angle of trunk rotation control 903 may be configured to display related historical information. The electronic device 100 displays an angle of trunk rotation history 906 in response to a user operation of touching the angle of trunk rotation control, and may display a historical angle control, a historical picture control, and a historical video control, and display a user interface shown in FIG. 9B.

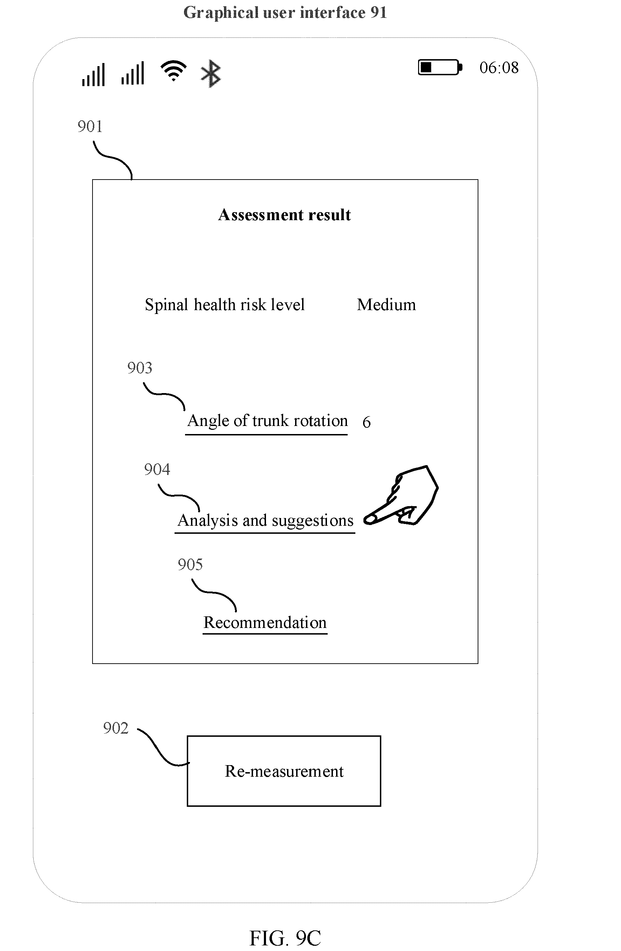

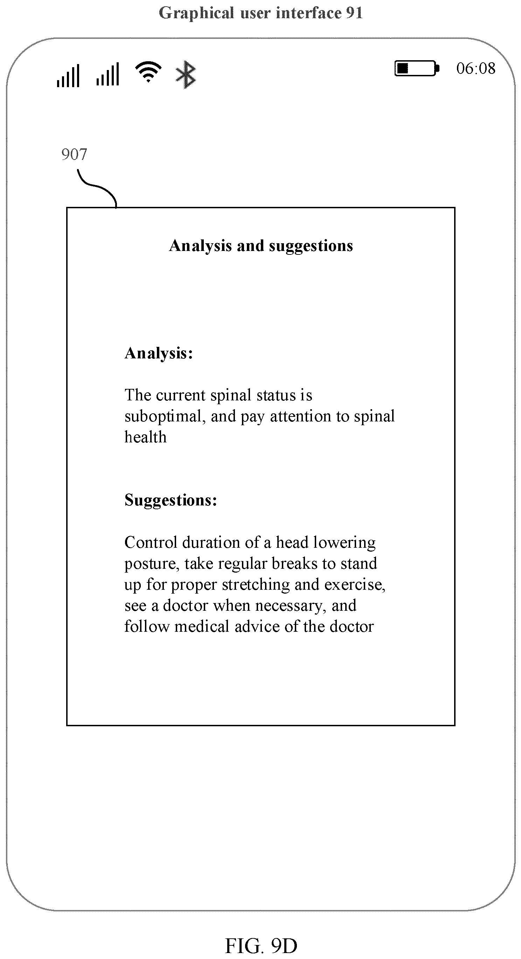

The analysis and suggestions control 904 may be configured to display a corresponding analysis and suggestions based on the spinal health risk level. As shown in FIG. 9C, in response to touching the analysis and suggestions control 904, the electronic device 100 displays an analysis and suggestions 907 corresponding to the current spinal health risk level, and displays a user interface shown in FIG. 9D.

In some implementations, specific content of the analysis and suggestions 907 may be a preset analysis and suggestions corresponding to the spinal health risk level, or may be an analysis and suggestions queried and updated through Internet searching. This is not limited herein.

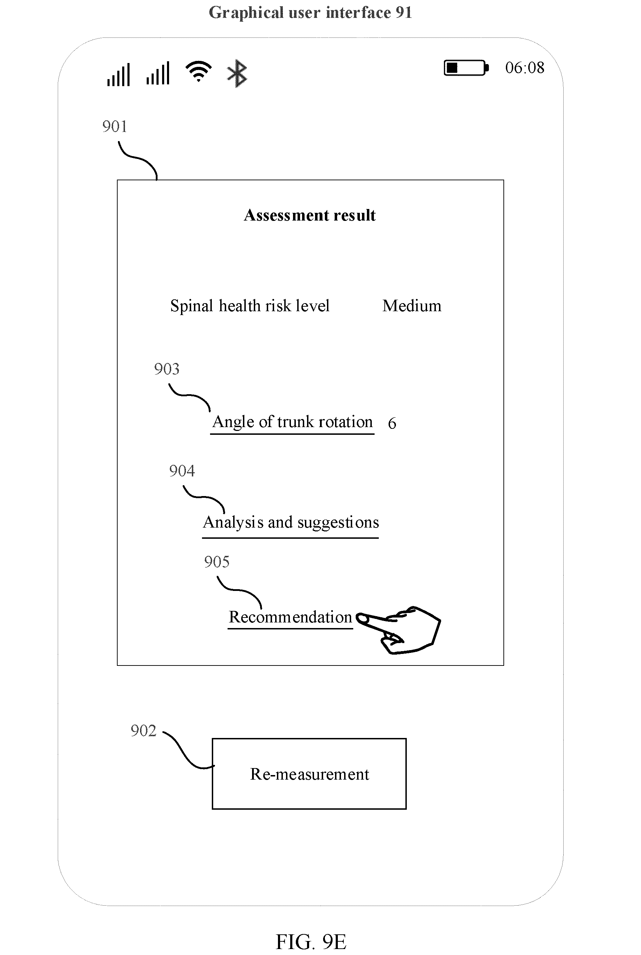

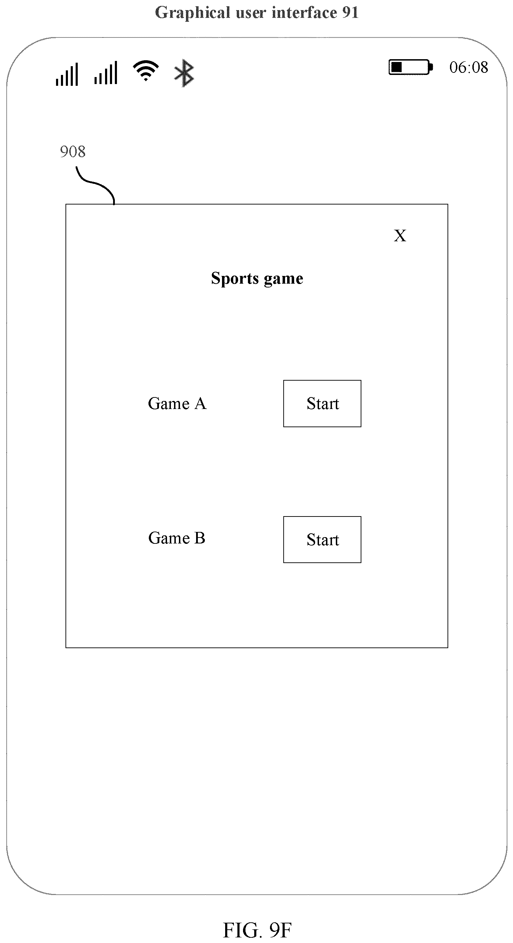

The recommendation control 905 may be configured to recommend a corresponding sports game based on the spinal health risk level. As shown in FIG. 9E, in response to touching the recommendation control 905, the electronic device 100 displays a sports game 908 recommended for the current spinal health risk level, and displays a user interface shown in FIG. 9F.

In some implementations, the sports game 908 may be a related sports game preset in the intelligent care application, or may be a related sports game in a system preset application or a game application. This is not limited herein. In some implementations, after displaying the recommended sports game, the electronic device 100 may start the game application through touching by the user, to play the sports game.

Based on the electronic device described in FIG. 2A to FIG. 2C and the user interfaces provided in the foregoing UI embodiments, the following describes in detail a spinal health risk assessment method according to an embodiment of this application.

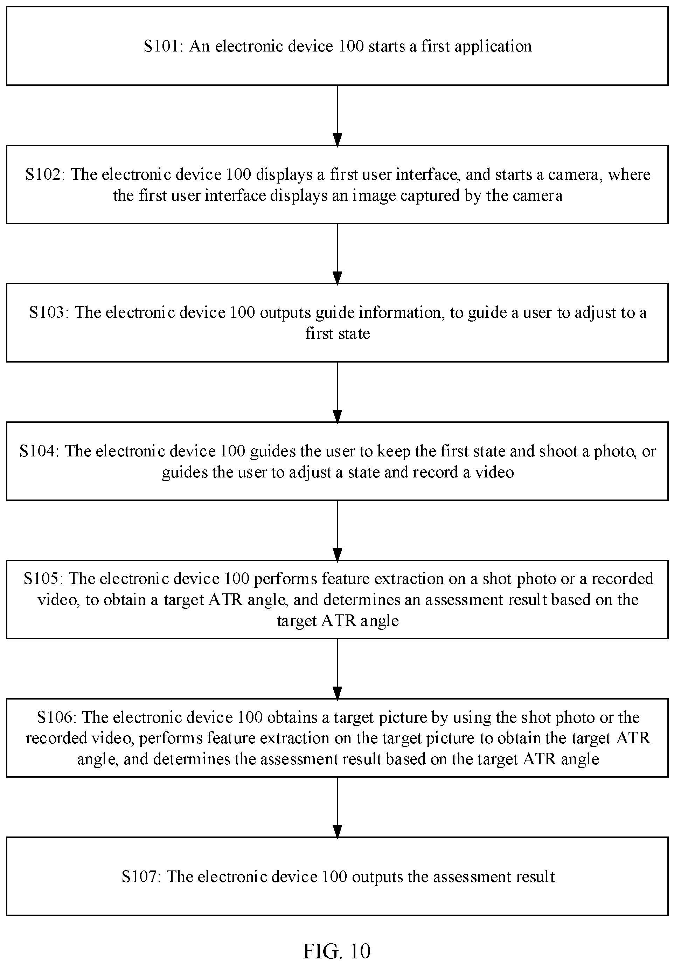

FIG. 10 shows an example of a procedure of a spinal health risk assessment method according to an embodiment of this application.

As shown in FIG. 10, the method may include the following steps.

S101: An electronic device 100 starts a first application.

The first application may include the "intelligent care application" mentioned in the foregoing embodiments. The first application may be a system application (for example, a camera application or another system application), or may be a third-party application.

The first application is installed in the electronic device 100, and is configured to: assess a spinal health risk, and output an assessment result of the spinal health risk. The electronic device 100 is supported in displaying a specific control on the display in response to receiving a user operation. The specific control may include a physical health assessment control. In addition, the "intelligent care application" supports the electronic device 100 in starting and performing an associated operation of physical health assessment after receiving a user operation performed on the control.

An implementation form of the specific control may be, for example, a text, a button, or an icon. This is not limited herein.

In some implementations of this application, the first application supports the electronic device 100 in starting and performing physical health assessment in response to a user operation, and outputting a target ATR angle.

In some other implementations of this application, the electronic device 100 may start the first application in response to a received user operation (for example, voice control, a tap operation, or a touch operation).

S102: The electronic device 100 displays a first user interface, and starts a camera, where the first user interface displays an image captured by the camera.

The electronic device 100 displays the first user interface in response to touching, by the user, an icon of the first application, where the first user interface is provided by the first application.

In some implementations, the electronic device 100 may directly display the first user interface in response to touching, by the user, the icon of the first application.

In some other implementations, the electronic device 100 displays a user interface shown in FIG. 4A in response to touching, by the user, the icon of the first application, and displays a user interface with a spinal health control shown in FIG. 4A in response to a touch operation of touching, by the user, the spinal health control. The electronic device 100 displays a user interface with precautions and a measurement start control shown in FIG. 4B in response to the touch operation of touching, by the user, the spinal health control, and displays the first user interface in response to a touch operation of touching, by the user, the measurement start control.

In some other implementations, the electronic device 100 displays a user interface with a physical health assessment control in response to touching, by the user, the icon of the first application, and displays a user interface with a spinal health control shown in FIG. 4A in response to a touch operation of touching, by the user, the physical health assessment control. The electronic device 100 displays a user interface with precautions and a measurement start control shown in FIG. 4B in response to the touch operation of touching, by the user, the spinal health control, and displays the first user interface in response to a touch operation of touching, by the user, the measurement start control.

In some implementations, when displaying the first user interface, the electronic device 100 may start a first camera or a second camera. The first camera may be a front-facing camera, and the second camera may be a rear-facing camera. For example, the electronic device 100 may start the first camera by default. After receiving an operation of touching, by the user, the camera switching control, the electronic device 100 switches the first camera to the second camera.

In some implementations, the first user interface may include a preview area, a status bar, an information prompt box, a standard guide block diagram, a bottom control, and the like.

The preview area may be configured to display the image captured by the camera. The electronic device 100 displays a real-time state of the user by using the preview area, and the user may adjust the state based on content displayed in the preview area. The preview area may include the standard guide block diagram. The preview area may further include the status bar, the information prompt box, the bottom control, and the like, or may not include the status bar, the information prompt box, the bottom control, and the like.

The information prompt box is used by the electronic device 100 to remind and guide the user, and after detecting that the user is in the first state, the electronic device 100 gives an instruction prompt to the user to start to shoot a photo or record a video, where the first state includes a first position and a first posture, a distance between the first position and a standard position is less than a first value, a difference between the first posture and a standard posture is less than a second value, the standard position means that a portrait of the user is in the standard guide block diagram, and the standard posture means that both legs keep apart at a distance the same as a shoulder width, a body is bent forward by 90 degrees, a back side faces the camera, or both palms are pressed together and center on both knees.

In some implementations, the electronic device 100 may alternatively prompt the user in another manner. For example, the electronic device 100 prompts the user by using voice information. This is not limited herein.

The standard guide block diagram may be dynamic or static. The dynamic standard guide block diagram means that a height and a width of the standard guide block diagram are properly adjusted based on data such as a height and a weight of a user, and the static standard guide block diagram means that the standard guide block diagram is displayed in a preset preview area. The standard guide block diagram is used to guide a state of the user.

A bottom menu bar may include a camera switching control, a start control, a shooting/recording switching control, and the like.

The camera switching control is configured to provide a function of switching between the front-facing camera and the rear-facing camera. For example, after receiving an operation of touching, by the user, the camera switching control, the electronic device 100 obtains the currently used first camera, and switches the first camera to the second camera. The first camera may be the front-facing camera, and the second camera may be the rear-facing camera.

The start control is configured to: after the electronic device 100 detects that the user is in the first state, prompt the user to start to shoot a photo or record a video.

This is not limited to the method for prompting, by using the start control, the user to start to shoot a photo or record a video. In some other implementations, when detecting that the user is in the first state, the electronic device 100 prompts, by using the information prompt box and/or voice information, the user to start to shoot a photo or record a video.

In some implementations, the start control may prompt, by using a color change, a form change, or the like, whether the user is in the first state. For example, when the electronic device 100 does not detect that the user is in the first state, the start control is displayed in red, and when the electronic device 100 detects that the user is in the first state, the start control is displayed in green.

In some implementations, the electronic device 100 starts to shoot a photo or record a video in response to an operation of touching, by the user, the start control, or in response to voice control of the user. In this case, the electronic device 100 may prompt, by using voice information, the user that "a photo is being shot" or "a video is being recorded", or may prompt the user by using the information prompt box. This is not limited herein.

The shooting/recording switching control is configured to provide a function of switching between photo shooting and video recording. The electronic device 100 switches between a photo shooting mode and a video recording mode in response to a user operation of touching the shooting/recording switching control. Specifically, when detecting that the intelligent care application is in the photo shooting mode, the electronic device 100 switches the intelligent care application to the video recording mode in response to a user operation of touching the shooting/recording switching control, and the user records a video during spinal health risk assessment.

In some implementations, in response to the operation of touching, by the user, the shooting/recording switching control 505, the electronic device 100 performs video recording after detecting that the user is in the first state and receiving that the user touches the start control.

In some other implementations, the electronic device 100 guides the user in response to touching, by the user, the measurement start control 404, to assess a spinal health risk level of the user after the user is in the first state.

S103: The electronic device 100 outputs guide information, to guide the user to adjust to the first state.

The guide information is used to prompt the user to adjust to the first state.

The first state includes the first position and the first posture. The distance between the first position and the standard position is less than the first value, and the difference between the first posture and the standard posture is less than the second value.

The standard position means that the portrait of the user is located at a center of the preview area and is located in the standard guide block diagram.

The standard posture includes any one or more of the following: both legs keep apart at a distance the same as a shoulder width, a body is bent forward by 90 degrees, a back side faces the camera, or both palms are pressed together and center on both knees.

In some implementations, the electronic device 100 outputs the guide information when starting to display the first user interface.

In some other implementations, the electronic device 100 outputs the guide information after detecting that the portrait exists in the image captured by the camera.

In some implementations, the electronic device 100 guides, by using the standard guide block diagram and the information prompt box, the user to adjust the state, so that the user adjusts to the first state. The standard guide block diagram in the first user interface is used to show the first position to the user, and the information prompt box is used to display: The user does not meet a part in the first posture. Text information in the information prompt box may include "The current portrait deviates. Adjust the portrait position", "Face away from the camera", "Bend forward by 90 degrees", and the like. The foregoing method makes it convenient for an assessor to help a to-be-assessed person in state adjustment.

In some other implementations, the electronic device 100 guides, by using a voice prompt, the user to adjust the state, so that the user adjusts to the first state, and prompts the user by using voice information. Voice information content includes "The current portrait deviates. Adjust the portrait position", "Face away from the camera", "Put both palms together", and the like. The foregoing method helps the to-be-assessed person to assess the spinal health risk alone by using the first application, and complete adjustment of the state of the to-be-assessed person independently.

In some other implementations, the electronic device 100 guides, by using the standard guide block diagram, the information prompt box, and the voice prompt, the user to adjust the state. The standard guide block diagram and voice information in the first user interface are used to show the first position of the user, and the information prompt box is used to display: The user does not meet a part in the first posture. In addition, the voice information is used to prompt that the user does not meet a part in the first posture. Text information in the information prompt box and voice information content may include "The current portrait deviates. Adjust the portrait position", "Face away from the camera", "Bend forward by 90 degrees", and the like. A combination of texts, pictures, and the voice prompt helps the user to learn from a plurality of perspectives, and adjust to the first state.

In some implementations, the electronic device 100 guides, by using the standard guide block diagram, the user to adjust to the first position, recognizes body node positions of the user in real time by using a bone node algorithm, and guides, based on a proportional relationship and a position relationship between the nodes, the user to adjust to the first state. In a process in which the electronic device 100 guides the user to adjust the state, the electronic device 100 guides the user through adjustment by using voice information and/or the information prompt box.

For example, with reference to FIG. 5C to FIG. 5E, when detecting that the portrait of the user deviates from the standard guide block diagram, the electronic device 100 reminds and guides the user by using voice information and/or text information in the information prompt box, so that the user adjusts a portrait position of the user to the first position.

The electronic device 100 recognizes the body node positions of the user in the portrait in real time by using the bone node algorithm. When detecting that a front side of the user faces the electronic device 100, the electronic device 100 guides, by using voice information and/or the information prompt box, the user to face the electronic device 100 with the back side.

The electronic device 100 recognizes the body node positions of the user in the portrait in real time by using the bone node algorithm, determines, by using the position relationship between the body nodes of the user after detecting that the back side of the user faces the electronic device 100, whether both legs of the user keep apart at a distance the same as a shoulder width, and detects, by using the proportional relationship between the body nodes of the user, whether the body of the user is bent forward by 90 degrees, whether both legs keep apart at a distance the same as a shoulder width, and whether both palms of the user are pressed together and center on both knees. If the electronic device 100 detects that the body of the user is not bent forward by 90 degrees and/or both palms are not in a state of being pressed together and centered on both knees, the electronic device 100 guides, by using voice information and/or the information prompt box, the user to adjust the hand posture to the first posture.

When the electronic device 100 detects that the user is in the first state, the start control is displayed in green, and the electronic device 100 may prompt, by using the start control, the user to start to shoot a photo, or the electronic device 100 may prompt, by using voice information, the user to start to shoot a photo.

Optionally, when obtaining that the portrait of the user is not completely displayed or is excessively large, the electronic device 100 guides, by using voice information and/or the information prompt box, the user to stay away from the electronic device 100.

Optionally, when detecting that the portrait of the user is not clearly displayed or the portrait is excessively small, the electronic device 100 guides, by using voice information and/or the information prompt box, the user to approach the electronic device 100.

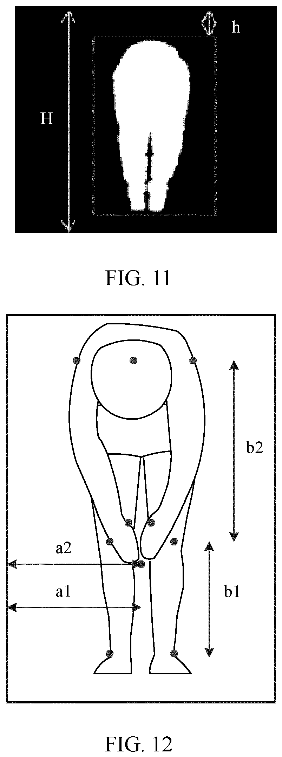

This is not limited to guiding, by using the voice information and/or the information prompt box, the user to approach the electronic device 100. In some other implementations of this application, the electronic device 100 may alternatively adjust the portrait of the user by performing proportional scaling processing on the size of the portrait. As shown in FIG. 11, the electronic device 100 selects the portrait by using a portrait segmentation algorithm, calculates a proportion (h/H) of the size of the portrait in the preview area, and adjusts a focal length, to make the size of the portrait moderate.

In some implementations, the electronic device 100 performs image analysis on the image captured by the camera, to determine whether the user is in the first state.

In some implementations, performing, by the electronic device 100, image analysis on the image captured by the camera includes recognizing the body node positions of the user in real time by using the bone node algorithm, detecting that the front side of the user faces the electronic device 100, and estimates a body forward flexion angle and a shoulder deviation based on the proportional relationship between the nodes. As shown in FIG. 12, the electronic device 100 obtains a half distance a1 of a width of the preview area of the portrait, a distance a2 from a hand tip to a left edge of the preview area, a distance b1 from a knee to an ankle, and a distance b2 from a shoulder to the tip of the hand in both hands, where a hand position is determined through a1/a2, and the body forward flexion angle is determined through b1/b2. When a1/a2 is between 0.9 and 1.1, and b1/b2 is between 0.6 and 0.8, the algorithm determines that a current posture is that the body is bent forward by 90 degrees and both palms are in a state of being pressed together and centered on both knees, and determines that the user is in the first state.

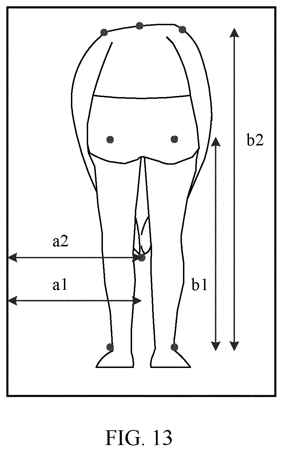

Optionally, performing, by the electronic device 100, image analysis on the image captured by the camera includes recognizing the body node positions of the user in real time by using the bone node algorithm, detecting that the back side of the user faces the electronic device 100, and estimates a body forward flexion angle and a shoulder deviation based on the proportional relationship between the nodes. As shown in FIG. 13, the electronic device 100 obtains a half distance a1 of a width of the preview area, a distance a2 from a hand tip in the portrait to a left edge of the preview area, a distance b1 from a hip to an ankle, and a distance b2 from a shoulder to the ankle, where a hand position is determined through a1/a2, and the body forward flexion angle is determined through b1/b2. When a1/a2 is between 0.9 and 1.1, and b1/b2 is between 0.6 and 0.8, the algorithm determines that a current posture is that the body is bent forward by 90 degrees and both palms are in a state of being pressed together and centered on both knees, and determines whether the user is in the first state.

S104: The electronic device 100 guides the user to keep the first state and take a photo, or guides the user to adjust the state and record a video.

In some implementations, the electronic device 100 guides the user to keep the first state. When it is detected that the user is in the first state, the start control is displayed in green. The electronic device 100 prompts, by using voice information and the start control, the user to start to shoot a photo. In response to a user operation of touching the start control and/or voice control, the electronic device prompts, by using the voice information and the start control, to keep the first state, completes photo shooting within a first preset time, and obtains a person image in which the user is in the first state through photo shooting, thereby saving an operation time of the user.

Specifically, the electronic device 100 may start to shoot a picture in response to a user operation of touching the start control and/or voice control, to shoot one or more pictures at a specific frequency.

Specifically, the electronic device 100 may further start to shoot a picture in response to a user operation of touching the start control and/or voice control, and shoot one or more pictures after receiving a touch operation of tapping by the user. For example, the user taps the start control once, and the electronic device 100 shoots one picture, and stops shooting a picture after a specific time or after a specific quantity of pictures are shot; or the user taps the start control once, and the electronic device shoots five pictures.

In some other implementations, when the electronic device 100 detects that the user is in the first state, the start control is displayed in green. The electronic device 100 prompts, by using voice information and the start control, the user to start to record a video. In response to a user operation of touching the start control and/or voice control, the electronic device prompts, by using the voice information and the start control, the user to change from an upright state to the first state, and completes video recording within a second preset time. Any posture from the upright state to the first state of the user is obtained through video recording, and body information of the user is obtained more comprehensively, to help subsequent obtaining of the target picture.

Specifically, the electronic device may further prompt, by using the voice information and the start control, the user to change from the first state to the upright state, and complete video recording within the second preset time.

Specifically, this is not limited to limiting recording duration of the video based on the second preset time. The electronic device 100 may alternatively limit recording of the video by using another method. For example, the electronic device 100 controls, in response to voice of the user, starting and ending of video recording. This is not limited herein.

S105: The electronic device 100 performs feature extraction on a shot photo or a recorded video, to obtain the target ATR angle, and determines the assessment result based on the target ATR angle.

The electronic device 100 performs feature extraction on the photo or the video: processing such as portrait segmentation, edge extraction, midline recognition, and tangent searching, and calculates the target ATR angle after feature extraction is completed, to determine a corresponding spinal health risk level.

The target ATR angle may include an ATR angle in a photo or a video frame, or may include a largest ATR angle in a plurality of ATR angles in a plurality of photos or video frames. The target ATR angle is used to determine the spinal health risk level and further determine a spinal health risk assessment result.

When a user posture in a photo or a video frame is bending forward by 90 degrees, in this case, a difference between a real-time ATR angle and the largest ATR angle is the smallest. Therefore, the ATR angle in the photo or the video frame is the target ATR angle.

A plurality of corresponding mapping relationships may be included between the target ATR angle and the spinal health risk level. For example, the mapping relationships may include but are not limited to: When the targetATR angle is at levels 0 to 4, the corresponding spinal health risk level is low; when the target ATR angle is at levels 5 to 7, the corresponding spinal health risk level is medium; and when the targetATR angle is at levels 8 to 10, the corresponding spinal health risk level is high.

The assessment result may be any one or more of the following: the target ATR angle and the spinal health risk level or score.

A larger target ATR angle corresponds to a higher spinal health risk of the user. The spinal health risk may be represented by the spinal health risk level, and the spinal health risk level may be low, medium, high, or the like. A higher spinal health risk level corresponds to a higher spinal health risk of the user. A higher score indicates a higher level, and corresponds to a higher spinal health risk of the user.

Optionally, in some other implementations, the electronic device 100 may determine a spinal health status by recognizing back skin textures, back Moire patterns, and the like. A larger difference between the back skin textures and healthy skin textures corresponds to a poorer spinal health status, and a larger spine line tortuosity of the back Moire patterns corresponds to a poor spinal health status.

Optionally, in S106, the electronic device 100 obtains the target picture by using the shot photo or the recorded video, performs feature extraction on the target picture to obtain the target ATR angle, and determines the assessment result based on the target ATR angle.

In some implementations, after the electronic device 100 guides the user to keep the first state and shoot a photo, the electronic device 100 selects a picture with a clear portrait from shot photos as the target picture.

In some other implementations, after the electronic device 100 guides the user to adjust the state and record the video, the electronic device 100 extracts key frames from the video, filters the key frames, and determines the target picture.

Optionally, the electronic device 100 extracts the key frames from the video at equal time intervals based on time information, where pictures corresponding to the key frames are target pictures.

Optionally, the electronic device 100 recognizes a body forward flexion angle of a portrait in the video, and pictures in which body forward flexion angles are 90 degrees, 75 degrees, 60 degrees, and 45 degrees are target pictures.

Optionally, the electronic device 100 extracts the key frames from the video at equal time intervals based on the time information, filters body forward flexion angles in the extracted key frames, and selects pictures in which body forward flexion angles are 90 degrees and 45 degrees as target pictures.

The target picture may be one picture, or may be a plurality of pictures. The target picture is used to determine the target ATR angle. The electronic device 100 may perform feature extraction on the target picture, determine a back tangent by using the target picture, further determine the target ATR angle, and determine the spinal health status based on the target ATR angle.

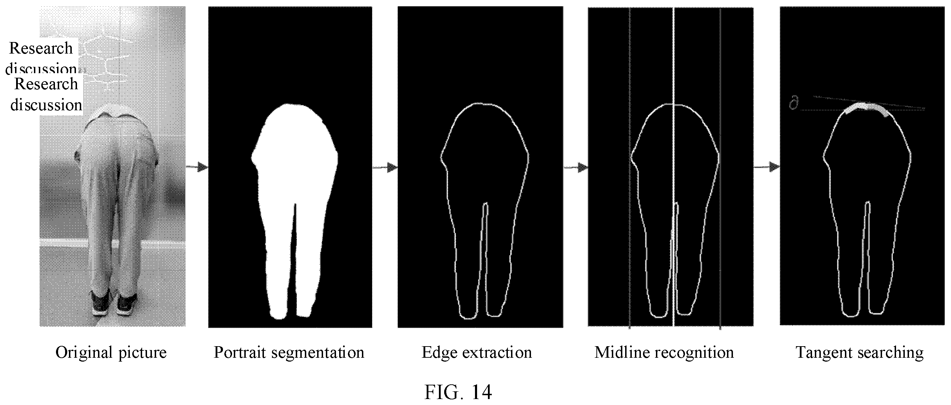

For example, with reference to FIG. 14, the electronic device 100 extracts a portrait part (portrait segmentation) from the target picture by using a deep neural network UNET, then determines edge information (edge extraction) of the portrait part, then estimates a human body midline position (midline recognition) based on positions on left and right sides of a portrait edge, and finally selects a point set on either of the left side and the right side of the midline, and searches each point set for two points that may be used as a back tangent (tangent search). The target ATR angle is calculated and determined based on the back tangents, and the spinal health status is determined based on the target ATR angle.

When the target picture is one picture, a user posture in the target picture is bending forward by 90 degrees, and a difference between an ATR angle in the target picture and the largest ATR angle is the smallest. In this case, the ATR angle in the picture is the target ATR angle.

S107: The electronic device 100 outputs the assessment result.

The assessment result indicates a spinal health status of the user.

An output form of outputting the assessment result by the electronic device 100 may include a plurality of forms, for example, may include but is not limited to: text display, picture display, voice broadcast, and the like.

In some implementations, not only the assessment result may be output, but also an angle of trunk rotation, an analysis and suggestions, a related recommendation, more content, and the like may be output.

The angle of trunk rotation control may be configured to display related historical information. The electronic device 100 displays an angle of trunk rotation history in response to a user operation of touching the angle of trunk rotation control, and may display a historical angle control, a historical picture control, and a historical video control, and display a user interface shown in FIG. 9B.

The analysis and suggestions may be used to display a corresponding analysis and suggestions based on the spinal health risk level. The analysis and suggestions include detailed analysis made for the spinal health risk level, and an exercise suggestion, a diet suggestion, a medical suggestion, a living habit suggestion, and the like. In response to touching the analysis and suggestions control, the electronic device 100 displays an analysis and suggestions corresponding to the current spinal health risk level, and displays a user interface shown in FIG. 9D.

In some implementations, specific content of the analysis and suggestions may be a preset analysis and suggestions corresponding to the spinal health risk level, or may be an analysis and suggestions queried and updated through Internet searching. This is not limited herein. For example, when the spinal health risk level is medium, it is analyzed that a current spinal health status is general, and it is recommended that a time of lowering the head be controlled, proper stretching be performed, and the like.

The related recommendation may be used to recommend a corresponding sports game based on the spinal health risk level. The related recommendation includes a sports game recommendation, a diet recommendation, and the like. A proper sports game is provided for the user through the related recommendation, to improve positiveness of improving the spinal health status by the user. In response to touching the recommendation control, the electronic device 100 displays a sports game recommended for the current spinal health risk level, and displays a user interface shown in FIG. 9F.

In some implementations, the sports game may be a related sports game preset in the intelligent care application, or may be a related sports game in a system preset application or a game application. This is not limited herein.

In some implementations, after displaying the recommended sports game, the electronic device 100 may start the game application through touching by the user, to play the sports game. When the user wants to play the sports game, the sports game may be directly started in the first application, to facilitate user operations.

The implementations of this application may be randomly combined to achieve different technical effects.

All or some of the foregoing embodiments may be implemented through software, hardware, firmware, or any combination thereof. When software is used to implement the embodiments, all or some of the embodiments may be implemented in a form of a computer program product. The computer program product includes one or more computer instructions. When the computer program instructions are loaded and executed on a computer, the procedures or functions according to this application are all or partially generated. The computer may be a general-purpose computer, a dedicated computer, a computer network, or another programmable apparatus. The computer instructions may be stored in a computer-readable storage medium, or transmitted from one computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, wireless, or microwaves) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

A person of ordinary skill in the art may understand that all or some of the procedures of the methods in the foregoing embodiments may be implemented by a computer program instructing related hardware. The program may be stored in the computer-readable storage medium. When the program is executed, the procedures in the foregoing method embodiments may be included. The foregoing storage medium includes any medium that can store program code, such as a ROM, a random access memory RAM, a magnetic disk, or an optical disc.

In conclusion, the foregoing is merely an embodiment of the technical solution of this application, but is not intended to limit the protection scope of this application. Any modification, equivalent replacement, improvement, or the like made based on the disclosure of this application shall fall within the protection scope of this application.

## Claims

1. A spinal health risk assessment method, wherein the method comprises:
starting, by an electronic device, a camera, and capturing an image by using the camera;
displaying, by the electronic device on a first user interface, the image captured by the camera;
outputting, by the electronic device, guide information, wherein the guide information is used to prompt a user to adjust to a first state;
after the image captured by the camera represents that the user is in the first state, starting, by the electronic device, to shoot a photo or record a video; and
determining and outputting, by the electronic device, an assessment result based on the photo or the video.

2. The method according to claim 1, wherein the guide information comprises an interface element displayed by the electronic device on the first user interface, and/or a voice instruction output by the electronic device.

3. The method according to claim 1 or 2, wherein the first state comprises a first position and a first posture;
a distance between the first position and a standard position is less than a first value, and a difference between the first posture and a standard posture is less than a second value; and
the standard posture comprises any one or more of the following: both legs keep apart at a distance the same as a shoulder width, a body is bent forward by 90 degrees, a back side faces the camera, or both palms are pressed together and center on both knees.

4. The method according to claim 3, wherein outputting, by the electronic device, the guide information specifically comprises:
displaying, by the electronic device, a guide box on the first user interface;
outputting, by the electronic device, first guide information, wherein the first guide information is used to prompt the user to adjust to the first position; and
outputting, by the electronic device, second guide information when the guide box displays a portrait captured by the camera, wherein the second guide information is used to prompt the user to adjust to the first posture.

5. The method according to claim 4, wherein before outputting, by the electronic device, the second guide information, the method further comprises:
adjusting, by the electronic device, a focal length of the camera based on a size of the portrait captured by the camera, and/or prompting the user to adjust a distance from the camera.

6. The method according to any one of claims 1 to 5, wherein after outputting, by the electronic device, the guide information, the method further comprises:
performing, by the electronic device, image analysis on the image captured by the camera, to determine whether the user is in the first state.

7. The method according to any one of claims 1 to 6, wherein after outputting, by the electronic device, the guide information, the method further comprises:
determining, by the electronic device, a first distance and a second distance in the portrait captured by the camera, wherein the first distance is a distance from a knee to an ankle, and the second distance is a distance from a shoulder to a tip of a hand in both hands, or the first distance is a distance from a hip to the ankle, and the second distance is a distance from the shoulder to the ankle; and
determining, by the electronic device, a body forward flexion angle of the user based on a ratio of the first distance to the second distance, wherein the body forward flexion angle of the user is used by the electronic device to determine whether the user is in the first state.

8. The method according to any one of claims 1 to 7, wherein determining and outputting, by the electronic device, the assessment result based on the photo or the video specifically comprises:
determining, by the electronic device, an angle of trunk rotation ATR of the user based on the photo or the video;
determining, by the electronic device, a spinal health status of the user based on the ATR; and
outputting, by the electronic device, the assessment result indicating the spinal health status of the user.

9. The method according to claim 8, wherein determining, by the electronic device, the angle of trunk rotation ATR of the user based on the photo or the video specifically comprises:
extracting, by the electronic device, a first portrait from the photo or the video;
determining, by the electronic device, a human body midline position in the first portrait;
determining, by the electronic device from edge feature points of the first portrait, a first tangent point and a second tangent point that are respectively located on a left side and a right side of the human body midline position;
determining, by the electronic device, a back tangent comprising the first tangent point and the second tangent point; and
determining, by the electronic device, the angle of trunk rotation ATR of the user, wherein the ATR is an included angle between the back tangent and a horizontal line.

10. The method according to any one of claims 1 to 9, wherein after the image captured by the camera represents that the user is in the first state, and before starting, by the electronic device, to shoot the photo, the method further comprises:
outputting, by the electronic device, first prompt information, wherein the first prompt information is used to prompt the user to keep the first state.

11. The method according to any one of claims 1 to 10, wherein a plurality of photos are shot by the electronic device, and after starting, by the electronic device, to shoot the photo, the method further comprises:
obtaining, by the electronic device through filtering from the plurality of shot photos, one or more photos in which the user is in the first state as a target picture, wherein the target picture is used to determine the assessment result.

12. The method according to any one of claims 1 to 9, wherein after the image captured by the camera represents that the user is in the first state, and before starting, by the electronic device, to record the video, the method further comprises:
outputting, by the electronic device, second prompt information, wherein the second prompt information is used to prompt the user to change from the first state to an upright state, or prompt the user to change from the upright state to the first state.

13. The method according to any one of claims 1 to 12, wherein after starting, by the electronic device, to record the video, the method further comprises:
extracting, by the electronic device in a manner of equal time intervals, a plurality of target photos from a plurality of frames of images comprised in the recorded video; or
analyzing, by the electronic device, a body forward flexion angle of the user in each frame of image in the recorded video, and extracting a plurality of target pictures from the video in an extraction manner of equal angular spacings, wherein
the target pictures are used to determine the assessment result.

14. The method according to any one of claims 1 to 13, wherein before starting, by the electronic device, to shoot the photo or record the video, the method further comprises:
receiving, by the electronic device, a first operation, wherein the first operation is used to trigger the electronic device to shoot the photo or record the video.

15. The method according to any one of claims 1 to 14, wherein
the electronic device outputs the guide information when starting to display the first user interface; or
the electronic device outputs the guide information after detecting that the portrait exists in the image captured by the camera.

16. The method according to any one of claims 1 to 15, wherein the assessment result comprises any one or more of the following: the ATR and a spinal health risk level, wherein a larger ATR indicates a higher spinal health risk level.

17. The method according to any one of claims 1 to 16, wherein determining and outputting, by the electronic device, the assessment result based on the photo or the video, the method further comprises:
outputting, by the electronic device, any one or more of the following: an exercise suggestion, a diet suggestion, a medical suggestion, and a living habit suggestion; and/or
outputting, by the electronic device, a recommendation result of a sports game.

18. The method according to any one of claims 1 to 17, wherein before starting, by the electronic device, the camera, and capturing the image by using the camera, the method further comprises:
displaying, by the electronic device, third prompt information, wherein the third prompt information comprises a posture requirement for the user and a wearing requirement for the user.

19. An electronic device, comprising a display, a memory, and one or more processors, wherein the display and the memory are coupled to the one or more processors, the memory is configured to store computer program code, the computer program code comprises computer instructions, and the one or more processors invoke the computer instructions to enable the electronic device to perform:
starting a camera, and capturing an image by using the camera;
displaying, on a first user interface, the image captured by the camera;
outputting guide information, wherein the guide information is used to prompt a user to adjust to a first state;
after the image captured by the camera represents that the user is in the first state, starting to shoot a photo or record a video; and
determining and outputting an assessment result based on the photo or the video.

20. The electronic device according to claim 19, wherein the one or more processors are configured to invoke the computer instructions to enable the electronic device to perform:
displaying an interface element on the first user interface, and/or outputting a voice instruction.

21. The electronic device according to claim 19 or 20, wherein the first state comprises a first position and a first posture;
a distance between the first position and a standard position is less than a first value, and a difference between the first posture and a standard posture is less than a second value; and
the standard posture comprises any one or more of the following: both legs keep apart at a distance the same as a shoulder width, a body is bent forward by 90 degrees, a back side faces the camera, or both palms are pressed together and center on both knees.

22. The electronic device according to claim 21, wherein the one or more processors are specifically configured to invoke the computer instructions to enable the electronic device to perform:
displaying a guide box on the first user interface;
outputting first guide information, wherein the first guide information is used to prompt the user to adjust to the first position; and
outputting second guide information when the guide box displays a portrait captured by the camera, wherein the second guide information is used to prompt the user to adjust to the first posture.

23. The electronic device according to any one of claims 19 to 22, wherein the one or more processors are specifically configured to invoke the computer instructions to enable the electronic device to perform:
performing image analysis on the image captured by the camera, to determine whether the user is in the first state.

24. The electronic device according to any one of claims 19 to 23, wherein the one or more processors are specifically configured to invoke the computer instructions to enable the electronic device to perform:
determining a first distance and a second distance in the portrait captured by the camera, wherein the first distance is a distance from a knee to an ankle, and the second distance is a distance from a shoulder to a tip of a hand in both hands, or the first distance is a distance from a hip to the ankle, and the second distance is a distance from the shoulder to the ankle; and
determining a body forward flexion angle of the user based on a ratio of the first distance to the second distance, wherein the body forward flexion angle of the user is used by the electronic device to determine whether the user is in the first state.

25. The electronic device according to any one of claims 19 to 24, wherein the one or more processors are further configured to invoke the computer instructions to enable the electronic device to perform:
determining an angle of trunk rotation ATR of the user based on the photo or the video;
determining a spinal health status of the user based on the ATR; and
outputting the assessment result indicating the spinal health status of the user.

26. The electronic device according to claim 25, wherein the one or more processors are configured to invoke the computer instructions to enable the electronic device to perform:
extracting a first portrait from the photo or the video;
determining a human body midline position in the first portrait;
determining, from edge feature points of the first portrait, a first tangent point and a second tangent point that are respectively located on a left side and a right side of the human body midline position;
determining a back tangent comprising the first tangent point and the second tangent point; and
determining the angle of trunk rotation ATR of the user, wherein the ATR is an included angle between the back tangent and a horizontal line.

27. The electronic device according to any one of claims 19 to 26, wherein the one or more processors are configured to invoke the computer instructions to enable the electronic device to perform:
outputting first prompt information, wherein the first prompt information is used to prompt the user to keep the first state.

28. The electronic device according to any one of claims 19 to 27, wherein the one or more processors are configured to invoke the computer instructions to enable the electronic device to perform:
obtaining, through filtering from the plurality of shot photos, one or more photos in which the user is in the first state as a target picture, wherein the target picture is used to determine the assessment result.

29. The electronic device according to any one of claims 19 to 28, wherein the one or more processors are configured to invoke the computer instructions to enable the electronic device to perform:
outputting second prompt information, wherein the second prompt information is used to prompt the user to change from the first state to an upright state, or prompt the user to change from the upright state to the first state.

30. The electronic device according to any one of claims 19 to 29, wherein the one or more processors are configured to invoke the computer instructions to enable the electronic device to perform:
extracting, in a manner of equal time intervals, a plurality of target photos from a plurality of frames of images comprised in the recorded video; or
analyzing a body forward flexion angle of the user in each frame of image in the recorded video, and extracting a plurality of target pictures from the video in an extraction manner of equal angular spacings, wherein
the target pictures are used to determine the assessment result.

31. The electronic device according to any one of claims 19 to 30, wherein the one or more processors are configured to invoke the computer instructions to enable the electronic device to perform:
receiving a first operation, wherein the first operation is used to trigger the electronic device to shoot the photo or record the video.

32. The electronic device according to any one of claims 19 to 31, wherein the one or more processors are configured to invoke the computer instructions to enable the electronic device to perform:
outputting the guide information when starting to display the first user interface; or
outputting the guide information after detecting that the portrait exists in the image captured by the camera.

33. The electronic device according to any one of claims 19 to 32, wherein the assessment result comprises any one or more of the following: the ATR and a spinal health risk level, wherein a larger ATR indicates a higher spinal health risk level.

34. The electronic device according to any one of claims 19 to 33, wherein the one or more processors are configured to invoke the computer instructions to enable the electronic device to perform:
outputting any one or more of the following: an exercise suggestion, a diet suggestion, a medical suggestion, and a living habit suggestion; and/or
outputting a recommendation result of a sports game.

35. The electronic device according to any one of claims 19 to 34, wherein the one or more processors are configured to invoke the computer instructions to enable the electronic device to perform:
displaying third prompt information, wherein the third prompt information comprises a posture requirement for the user and a wearing requirement for the user.

36. A computer-readable storage medium, comprising instructions, wherein when the instructions are run on an electronic device, the electronic device is enabled to perform the method according to any one of claims 1 to 18.

37. A computer program product, wherein when the computer program product is run on a computer, the computer is enabled to perform the method according to any one of claims 1 to 18.
